# EUROPEAN PATENT APPLICATION

(11) **EP 4 744 623 A2**
(43) Date of publication of application: **20.05.2026**
(21) Application number: 25223853.0
(22) Date of filing: 13.09.2022
(51) Int. Cl.: A61F 2/24

(54) **DEVICES AND SYSTEMS FOR DOCKING A HEART VALVE**

(30) Priority: 17.09.2021 US 202163245721 P; 09.08.2022 US 202263370870 P
(62) Divisional of application: 22783194.8
(71) Applicant: Edwards Lifesciences Corporation, Irvine, CA 92614 (US)
(72) Inventor: CHEN, Jensen, Irvine, CA 92614 (US); RODRIGUEZ, Alison Louise, Irvine, CA 92614 (US); LEE, Walter, Irvine, CA 92614 (US); SCHAFFER, Andrew Paul, Irvine, CA 92614 (US)
(74) Representative: Eisenführ Speiser

(57) **Abstract**

A docking station frame for a medical device includes a plurality of strut portions extending from a proximal end to a distal end and forming a plurality of cells.

## Description

### RELATED APPLICATION

The present application claims priority to and all benefit of U.S. Provisional Patent Application Ser. No. 63/245,721, filed on September 17, 2021, and U.S. Provisional Patent Application Ser. No. 63/370,870, filed on August 9, 2022, both of which are incorporated herein by reference in their entirety.

### FIELD OF THE INVENTION

The present invention relates to heart valves and, in particular, docking stations/stents, delivery systems, and methods for use in implanting a heart valve, e.g., a transcatheter heart valve ("THV").

### BACKGROUND OF THE INVENTION

Prosthetic heart valves can be used to treat cardiac valvular disorders. The native heart valves (the aortic, pulmonary, tricuspid, and mitral valves) serve critical functions in assuring the forward flow of an adequate supply of blood through the cardiovascular system. These heart valves can be rendered less effective by congenital, inflammatory, or infectious conditions. Such conditions can eventually lead to serious cardiovascular compromise or death. For many years, the definitive treatment for such disorders was the surgical repair or replacement of the valve during open heart surgery.

A transcatheter technique can also be used for introducing and implanting a prosthetic heart valve using a flexible catheter in a manner that is less invasive than open heart surgery. In this technique, a prosthetic valve can be mounted in a crimped state on the end portion of a flexible catheter and advanced through a blood vessel of the patient until the valve reaches the implantation site. The valve at the catheter tip can then be expanded to its functional size at the site of the defective native valve, such as by inflating a balloon on which the valve is mounted. Alternatively, the valve can have a resilient, self-expanding stent or frame that expands the valve to its functional size when it is advanced from a delivery sheath at the distal end of the catheter.

Transcatheter heart valves (THVs) can be appropriately sized to be placed inside most native aortic valves. However, with larger native valves, blood vessels, and grafts, aortic transcatheter valves might be too small to secure into the larger implantation or deployment site. In this case, the transcatheter valve may not be large enough to sufficiently expand inside the native valve or other implantation or deployment site to be secured in place.

Replacing the pulmonary valve, which is sometimes referred to as the pulmonic valve, presents significant challenges. The geometry of the pulmonary artery can vary greatly from patient to patient. Typically, the pulmonary artery outflow tract after corrective surgery is too wide for effective placement of a prosthetic heart valve.

### SUMMARY

This summary is meant to provide examples and is not intended to be limiting of the scope of the invention in any way. For example, any feature included in an example of this summary is not required by the claims, unless the claims explicitly recite the feature. The description discloses exemplary embodiments of expandable docking stations for an expandable valve. The docking stations can be constructed in a variety of ways.

In some implementations, a docking station frame for a medical device includes a plurality of strut portions extending from a proximal end to a distal end and forming a plurality of cells.

In some implementations, when the docking station frame is in an expanded, unconstrained condition, the plurality of strut portions form a substantially continuous curved longitudinal profile having outer radial retaining portions at the proximal and distal ends and an inner radial central portion defining a valve seat.

In some implementations, the plurality of strut portions comprise a plurality of circumferential strut portions axially spaced to define a plurality of rows of the plurality of cells, and a plurality of longitudinal strut portions circumferentially spaced and joined with the plurality of circumferential strut to define the cells of the plurality of cells in each of the plurality of rows.

In some implementations, the plurality of circumferential strut portions comprises at least five circumferential strut portions defining at least four rows of the plurality of cells.

In some implementations, wherein the plurality of longitudinal strut portions comprises at least twelve longitudinal strut portions defining at least twelve cells in each of the plurality of rows.

In some implementations, the plurality of circumferential strut portions comprises undulating strut portions.

In some implementations, the parallel undulating strut portions are substantially parallel and shaped such that each of the plurality of cells is substantially herringbone shaped.

In some implementations, a distal one of the parallel undulating strut portions defines the outer radial retaining portion at the distal end, and a proximal one of the parallel undulating strut portions defines the outer radial retaining portion at the proximal end.

In some implementations, each of the plurality of undulating strut portions includes proximal portions joined with a corresponding one of the plurality of longitudinal strut portions, and distal portions unattached from the plurality of longitudinal strut portions.

In some implementations, when the docking station frame is in the expanded, unconstrained condition, the waist has a diameter of about 27 mm and is expandable to accommodate a valve having a diameter of about 29 mm.

In some implementations, when the docking station frame is in the expanded, unconstrained condition, the docking station frame has a length between about 35 mm and about 40 mm.

In some implementations, when the docking station frame is in the expanded, unconstrained condition, the retaining portions extend at an angle between about 30° and about 45° with respect to a central longitudinal axis of the docking station frame.

In some implementations, when the docking station frame is in the expanded, unconstrained condition, the waist portion is substantially cylindrical.

In some implementations, when the docking station frame is in the expanded, unconstrained condition, the retaining portions have a radius of curvature of between about 14 mm and about 20 mm.

In some implementations, when the docking station frame is in the expanded, unconstrained condition, the inner radial central portion is contoured to provide a valve seat having a length of at least about 8 mm for a valve having a deployed outer diameter of 29 mm.

In some implementations, the docking station frame is configured to be crimped to a diameter of no greater than about 5.4 mm.

In some implementations, when the docking station frame is in an expanded, unconstrained condition, the plurality of strut portions form outer radial retaining portions at the proximal and distal ends extending at an angle between about 30° and about 45° with respect to a central longitudinal axis of the docking station frame.

In some implementations, when the docking station frame is in the expanded, unconstrained condition, the inner radial central portion is substantially cylindrical.

In some implementations, when the docking station frame is in an expanded, unconstrained condition, the plurality of strut portions form a curved longitudinal profile having outer radial retaining portions at the proximal and distal ends and an inner radial central portion defining a valve seat, the inner radial central portion being substantially cylindrical and the outer radial retaining portions each having a radius of curvature between about 14 mm and about 20 mm.

In some implementations, when the docking station frame is in the expanded, unconstrained condition, the retaining portions have a maximum diameter of about 44 mm.

In some implementations, a docking station for a medical device includes the docking station frame and an impermeable material attached to the docking station frame.

In some implementations, the impermeable material is attached to the valve seat to provide a seal between the valve seat and a valve installed in the valve seat.

In some implementations, the impermeable material is attached to a medial portion of the docking station frame between the inner radial central portion and at least one of the outer radial retaining portions to provide a medial sealing portion for sealing with a deployment site in a circulatory system when the docking station is deployed at the deployment site.

In some implementations, a system includes a delivery catheter including a tube, and the docking station disposed in the tube.

In some implementations, a docking station for a prosthetic valve includes an expandable docking station frame including a plurality of struts extending from a first end to a second end of the docking station frame, with the expandable frame further defining a valve seat for securing a prosthetic valve to the expandable frame.

In some implementations, the first end of the docking station frame defines a retaining portion expandable radially outward to engage an inner surface of a circulatory system at a deployed position over a range of sizes of expansion.

In some implementations, a plurality of radiopaque markers are disposed around the first end of the docking station frame.

In some implementations, the first end of the docking station frame includes an endmost zigzag shaped portion of the docking station frame, alternating between outer axial apices and inner axial junctions.

In some implementations, each of the plurality of radiopaque markers is located between the inner axial junctions and the outer axial apices.

In some implementations, each of the plurality of radiopaque markers is disposed on a corresponding one of the plurality of the outer axial apices.

In some implementations, each of the plurality of radiopaque markers is attached to the corresponding one of the plurality of the outer axial apices.

In some implementations, the docking station frame includes a plurality of integrally formed marker seating portions each retaining one of the plurality of radiopaque markers therein.

In some implementations, the docking station includes an impermeable material attached to the docking station frame and having a first end portion disposed at a first end of the docking station frame, wherein each of the plurality of radiopaque markers is affixed to the first end portion of the impermeable material.

In some implementations, each of the plurality of radiopaque markers is integrally formed with the first end of the docking station frame.

In some implementations, the plurality of radiopaque markers comprises enlarged junctions between the plurality of struts.

In some implementations, the docking station includes a second plurality of radiopaque markers disposed around a second axial location of the docking station frame, axially spaced from the first end of the docking station frame.

In some implementations, the second plurality of radiopaque markers is disposed around the valve seat.

In some implementations, the second plurality of radiopaque markers isdisposed around the second end of the docking station frame.

In some implementations, the second plurality of radiopaque markers is disposed between outer axial apices and inner axial junctions of the second end of the docking station frame.

In some implementations, the second plurality of radiopaque markers is disposed on a plurality of the outer axial apices of the second end of the docking station frame.

In some implementations, the second plurality of radiopaque markers is attached to the plurality of the outer axial apices of the second end of the docking station frame.

In some implementations, the docking station frame includes a second plurality of integrally formed marker seating portions each retaining one of the second plurality of radiopaque markers therein.

In some implementations, the docking station further comprises an impermeable material attached to the docking station frame, wherein each of the second plurality of radiopaque markers is affixed to the impermeable material.

In some implementations, each of the second plurality of radiopaque markers is integrally formed with the docking station frame.

In some implementations, the second plurality of radiopaque markers comprises enlarged junctions between the plurality of struts.

In some implementations, each of the first plurality of radiopaque markers has a first configuration, and each of the second plurality of radiopaque markers has a second configuration different from the first configuration.

In some implementations, the second configuration differs from the first configuration in at least one of: marker size, marker shape, marker orientation, and distance between adjacent markers.

In some implementations, the valve seat is disposed on a medial portion of the docking station frame between the first and second ends.

In some implementations, the docking station frame defines a concave profile extending from the first and second ends to a narrower cylindrical or shallow concave waist portion.

In some implementations, the docking station frame defines a cylindrical outer portion, with the valve seat being offset radially inward from the outer portion.

In some implementations, the docking station frame includes at least one radially outward extending flanged end portion.

In some implementations, the docking station frame includes convex profiled first and second end portions and a concave waist portion defining the valve seat.

In some implementations, the docking station frame includes flared first and second end portions and a concave waist portion defining the valve seat.

In some implementations, in a method of installing a prosthetic valve in a vessel, an expandable docking station frame, such as, for example, any of the docking station frames described herein, is provided in a compressed condition within a first tube. The first tube is inserted into the vessel. The docking station frame is deployed from the first tube, and the docking station frame is expanded at a target location within the vessel, such that a first end of the docking station frame expands radially outward of the valve seat to engage an inner surface of the vessel to retain the docking station frame at the target location. The first tube is withdrawn from the vessel. An expandable prosthetic valve is provided in a compressed condition within a second tube. The second tube is inserted into the vessel, and a terminal end of the second tube is extended into the first end of the expanded docking station frame. The expandable prosthetic valve is deployed from the terminal end of the second tube, and the prosthetic valve is expanded into seating engagement with a valve seat of the expanded docking station frame.

In some implementations, a positional arrangement of the first end of the expanded docking station frame is visually confirmed by visually identifying locations of a plurality of radiopaque markers disposed around the first end of the docking station frame.

In some implementations, the docking station further comprises a second plurality of radiopaque markers disposed around a second axial location of the docking station frame, axially spaced from the first end of the docking station frame.

In some implementations, deploying the docking station frame from the first tube and expanding the docking station frame at the target location within the vessel comprises partially deploying the docking station frame from the first tube and visually confirming locations of the second plurality of radiopaque markers at the target location before fully deploying the docking station frame the docking station frame from the first tube.

In some implementations, the second plurality of radiopaque markers are disposed around the valve seat, and deploying the expandable prosthetic valve from the terminal end of the second tube comprises visually confirming locations of the second plurality of radiopaque markers in alignment with the expandable prosthetic valve before fully deploying the expandable prosthetic valve.

In some implementations, at least one of the prosthetic valve and the second tube terminal end includes at least one radiopaque marker, wherein visually confirming the locations of the second plurality of radiopaque markers in alignment with the expandable prosthetic valve comprises visually confirming the locations of the second plurality of radiopaque markers in alignment with the at least one radiopaque marker.

The above method(s) can be performed on a living animal or on a simulation, such as on a cadaver, cadaver heart, simulator (e.g., with the body parts, heart, leaflet, tissue, etc. being simulated), etc.

Various features as described elsewhere in this disclosure can be included in the examples summarized here and various methods and steps for using the examples and features can be used, including as described elsewhere herein.

Further understanding of the nature and advantages of the disclosed inventions can be obtained from the following description and claims, particularly when considered in conjunction with the accompanying drawings in which like parts bear like reference numerals.

### BRIEF DESCRIPTION OF THE DRAWINGS

To further clarify various aspects of embodiments of the present disclosure, a more particular description of the certain embodiments will be made by reference to various aspects of the appended drawings. It is appreciated that these drawings depict only typical embodiments of the present disclosure and are therefore not to be considered limiting of the scope of the disclosure. Moreover, while the drawings may be drawn to scale for some embodiments, the drawings are not necessarily drawn to scale for all embodiments. Embodiments of the present disclosure will be described and explained with additional specificity and detail through the use of the accompanying drawings.
FIG. 1A is a cutaway view of the human heart in a diastolic phase;
FIG. 1B is a cutaway view of the human heart in a systolic phase;
FIGS. 2A and 2B are sectional views of pulmonary arteries illustrating that pulmonary arteries can have a variety of different shapes and sizes;
FIGS. 3A and 3B are perspective views of pulmonary arteries illustrating that pulmonary arteries can have a variety of different shapes and sizes;
FIG. 4A is a schematic illustration of a compressed docking station being positioned in a circulatory system;
FIG. 4B is a schematic illustration of the docking station of FIG. 4A expanded to set the position of the docking station in the circulatory system;
FIG. 4C is a schematic illustration of an expandable transcatheter heart valve being positioned in the docking station illustrated by FIG. 4B;
FIG. 4D is a schematic illustration of the transcatheter heart valve of FIG. 4C expanded to set the position of the heart valve in the docking station;
FIG. 5A is a cutaway view of the human heart in a systolic phase with a docking station deployed in a pulmonary artery;
FIG. 5B is a cutaway view of the human heart in a systolic phase with a docking station and transcatheter heart valve deployed in a pulmonary artery;
FIG. 6A is an enlarged schematic illustration of the docking station and transcatheter heart valve of FIG. 5B when the heart is in the systolic phase;
FIG. 6B is a view taken in the direction indicated by lines 6B-6B in FIG. 6A;
FIG. 7 is a cutaway view of the human heart in a diastolic phase with a docking station and transcatheter heart valve deployed in a pulmonary artery;
FIG. 8A is an enlarged schematic illustration of the docking station and transcatheter heart valve of FIG. 7 when the heart is in the diastolic phase;
FIG. 8B is a view taken in the direction indicated by lines 8B-8B in FIG. 8A;
FIG. 9A is a side view of an exemplary embodiment of a frame of a docking station;
FIG. 9B illustrates a side profile of the frame of FIG. 9A;
FIG. 9C is an end view of the frame of FIG. 9A;
FIG. 9D is a perspective view of the frame of FIG. 9A;
FIG. 10 is a side view of an exemplary embodiment of a docking station having an impermeable material attached to an expandable frame;
FIGS. 11A and 11B schematically illustrate side profiles of the docking station illustrated by FIG. 10 when implanted in different size vessels of the circulatory system;
FIG. 12 is a sectional view illustrating a side profile of an exemplary embodiment of a docking station placed in a pulmonary artery and a schematically illustrated valve placed in the docking station;
FIG. 13A is a cutaway view of the human heart in a systolic phase with a docking station being deployed in a pulmonary artery;
FIG. 13B is a cutaway view of the human heart in a systolic phase with a docking station deployed in a pulmonary artery;
FIG. 13C is a cutaway view of the human heart in a systolic phase with a docking station and transcatheter heart valve deployed in a pulmonary artery;
FIGS. 14 - 17, and 18A - 18C illustrate examples of valve types that can be deployed in a docking station, e.g., one of the docking stations described or depicted herein;
FIG. 19A is a side view of a docking station with a frame and an impermeable material according to one embodiment;
FIG. 19B is a side view of a docking station with a frame and an impermeable material according to another embodiment;
FIG. 19C is a side view of a docking station with a frame and an impermeable material according to another embodiment;
FIG. 19D is a side view of a docking station with a frame and an impermeable material according to another embodiment; and
FIG. 20 is a perspective view of a docking station including an hourglass shaped frame and attached impermeable sealing material;
FIG. 21 is a partial view of an exemplary expandable frame, showing exemplary first end, second end, and central cells of the expandable frame;
FIG. 21A is a cross-sectional view of a first end portion of a longitudinal strut of the expandable frame of FIG. 21, taken along the plane indicated by lines 21A-21A of FIG. 21;
FIG. 21B is a cross-sectional view of a second end portion of a longitudinal strut of the expandable frame of FIG. 21, taken along the plane indicated by lines 21B-21B of FIG. 21;
FIG. 21C is a cross-sectional view of a central portion of a longitudinal strut of the expandable frame of FIG. 21, taken along the plane indicated by lines 21C-21C of FIG. 21;
FIG. 21D is a cross-sectional view of a first end portion of a circumferential strut of the expandable frame of FIG. 21, taken along the plane indicated by lines 21D-21D of FIG. 21;
FIG. 21E is a cross-sectional view of a second end portion of a circumferential strut of the expandable frame of FIG. 21, taken along the plane indicated by lines 21E-21E of FIG. 21;
FIG. 21F is a cross-sectional view of a central portion of a circumferential strut of the expandable frame of FIG. 21, taken along the plane indicated by lines 21F-21F of FIG. 21;
FIG. 22 is a side view of an exemplary docking station frame having a concave profile extending from flared end portions to a narrower waist portion;
FIG. 22A is a side view of an exemplary docking station frame having a cylindrical shaped outer frame portion and a radially inward offset valve seat portion;
FIG. 22B is a side view of another exemplary docking station frame having a cylindrical shaped outer frame portion and a radially inward offset valve seat portion;
FIG. 22C is a side view of an exemplary docking station frame having a radially outward extending flexible flanged portion extending from one end of an elongated cylindrical portion;
FIG. 22D is a side view of an exemplary docking station frame having radially outward extending flexible flanged portions extending from both ends of an elongated cylindrical portion;
FIG. 22E is a side view of an exemplary docking station frame having an hourglass shaped profile with convex end portions;
FIG. 22F is a side view of an exemplary docking station frame having an hourglass shaped profile with flared end portions;
FIG. 23A is a schematic side cross-sectional view of an exemplary vessel engagement profile of an installed docking station, with radial gaps between the retaining portions and the sealing portions, and a radial gap around the docking station waist portion;
FIG. 23B is a schematic side cross-sectional view of another exemplary vessel engagement profile of an installed docking station, with a concave profile of the docking station providing a radial gap around the docking station waist portion;
FIG. 23C is a schematic side cross-sectional view of another exemplary vessel engagement profile of an installed docking station, with a concave profile of the docking station in continuous contact with the vessel inner surface;
FIG. 23D is a schematic side cross-sectional view of another exemplary vessel engagement profile of an installed docking station, with a cylindrical profile of the docking station in continuous contact with a substantially cylindrical vessel inner surface;
FIG. 23E is a schematic side cross-sectional view of another exemplary vessel engagement profile of an installed docking station, with a flexible cylindrical profile of the docking station in continuous contact with a non-uniform vessel inner surface;
FIG. 23F is a schematic side cross-sectional view of another exemplary vessel engagement profile of an installed docking station, with tangential engagement at end portions and a radial gap around a waist portion of the docking station;
FIG. 24A is a schematic side cross-sectional view of an exemplary vessel engagement profile of an installed docking station, showing an end portion of the docking station frame deformed radially inward of a central opening of the docking station frame;
FIG. 24B is a schematic end view of the installed docking station of FIG. 24A;
FIG. 25A is a front perspective view of a radiopaque marker according to one embodiment;
FIG. 25B is a front perspective view of a radiopaque marker according to another embodiment;
FIG. 25C is a front perspective view of a radiopaque marker according to another embodiment;
FIG. 25D is a front perspective view of a radiopaque marker according to another embodiment;
FIG. 26 is a side view of an exemplary docking station frame having a concave profile extending from flared end portions to a narrower waist portion, with radiopaque markers affixed to a first end of the frame;
FIG. 26A is a side view of an exemplary docking station frame having a cylindrical shaped outer frame portion and a radially inward offset valve seat portion, with radiopaque markers affixed to a first end of the frame;
FIG. 26B is a side view of another exemplary docking station frame having a cylindrical shaped outer frame portion and a radially inward offset valve seat portion, with radiopaque markers affixed to a first end of the frame;
FIG. 26C is a side view of an exemplary docking station frame having a radially outward extending flexible flanged portion extending from one end of an elongated cylindrical portion, with radiopaque markers affixed to a first end of the frame;
FIG. 26D is a side view of an exemplary docking station frame having radially outward extending flexible flanged portions extending from both ends of an elongated cylindrical portion, with radiopaque markers affixed to a first end of the frame;
FIG. 26E is a side view of an exemplary docking station frame having an hourglass shaped profile with convex end portions, with radiopaque markers affixed to a first end of the frame;
FIG. 26F is a side view of an exemplary docking station frame having an hourglass shaped profile with flared end portions, with radiopaque markers affixed to a first end of the frame;
FIG. 27 is a side view of an exemplary docking station frame having a concave profile extending from flared end portions to a narrower waist portion, with radiopaque markers affixed to a material secured to a first end of the frame;
FIG. 27A is a side view of an exemplary docking station frame having a cylindrical shaped outer frame portion and a radially inward offset valve seat portion, with radiopaque markers affixed to a material secured to a first end of the frame;
FIG. 27B is a side view of another exemplary docking station frame having a cylindrical shaped outer frame portion and a radially inward offset valve seat portion, with radiopaque markers affixed to a material secured to a first end of the frame;
FIG. 27C is a side view of an exemplary docking station frame having a radially outward extending flexible flanged portion extending from one end of an elongated cylindrical portion, with radiopaque markers affixed to a material secured to a first end of the frame;
FIG. 27D is a side view of an exemplary docking station frame having radially outward extending flexible flanged portions extending from both ends of an elongated cylindrical portion, with radiopaque markers affixed to a material secured to a first end of the frame;
FIG. 27E is a side view of an exemplary docking station frame having an hourglass shaped profile with convex end portions, with radiopaque markers affixed to a material secured to a first end of the frame;
FIG. 27F is a side view of an exemplary docking station frame having an hourglass shaped profile with flared end portions, with radiopaque markers affixed to a material secured to a first end of the frame;
FIG. 28A is a cutaway view of the human heart in a systolic phase with a docking station being deployed in a pulmonary artery;
FIG. 28B is a cutaway view of the human heart in a systolic phase with a docking station deployed in a pulmonary artery;
FIG. 28C is a cutaway view of the human heart in a systolic phase with a docking station deployed in a pulmonary artery and a transcatheter heart valve being deployed in the pulmonary artery; and
FIG. 28D is a cutaway view of the human heart in a systolic phase with a docking station deployed in a pulmonary artery and a transcatheter heart valve being deployed in the pulmonary artery.

### DETAILED DESCRIPTION

The following description refers to the accompanying drawings, which illustrate specific embodiments of the invention. Other embodiments having different structures and operation do not depart from the scope of the present invention. Exemplary embodiments of the present disclosure are directed to devices and methods for providing a docking station or landing zone for a transcatheter heart valve ("THV"). In some exemplary embodiments, docking stations for THVs are illustrated as being used within the pulmonary artery, although the docking stations can be used in other areas of the anatomy, heart, or vasculature, such as the superior vena cava or the inferior vena cava. Further, the techniques and methods herein can be performed on a living animal or on a simulation, such as on a cadaver, cadaver heart, simulator (e.g., with the body parts, heart, tissue, etc. being simulated), etc. The docking stations described herein can be configured to compensate for the deployed THV being smaller than the space (e.g., anatomy/vasculature, etc.) in which it is to be placed.

It should be noted that various embodiments of docking stations and systems for delivery and implant are disclosed herein, and any combination of these options can be made unless specifically excluded. For example, any of the docking stations devices disclosed, can be used with any type of valve, and/or any delivery system, even if a specific combination is not explicitly described. Likewise, the different constructions of docking stations and valves can be mixed and matched, such as by combining any docking station type/feature, valve type/feature, tissue cover, etc., even if not explicitly disclosed. In short, individual components of the disclosed systems can be combined unless mutually exclusive or otherwise physically impossible.

For the sake of uniformity, in these figures and others in the application the docking stations are depicted such that the pulmonary bifurcation end is up, while the ventricular end is down. These directions may also be referred to as "distal" as a synonym for up or the pulmonary bifurcation end, and "proximal" as a synonym for down or the ventricular end, which are terms relative to the physician's perspective.

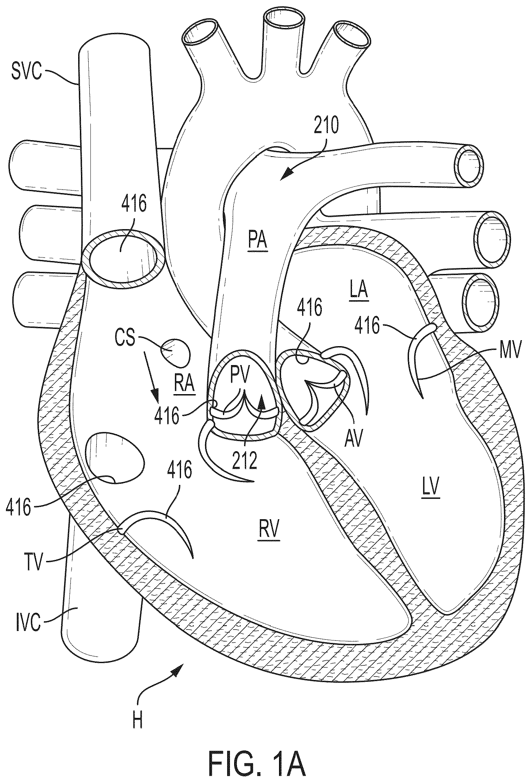

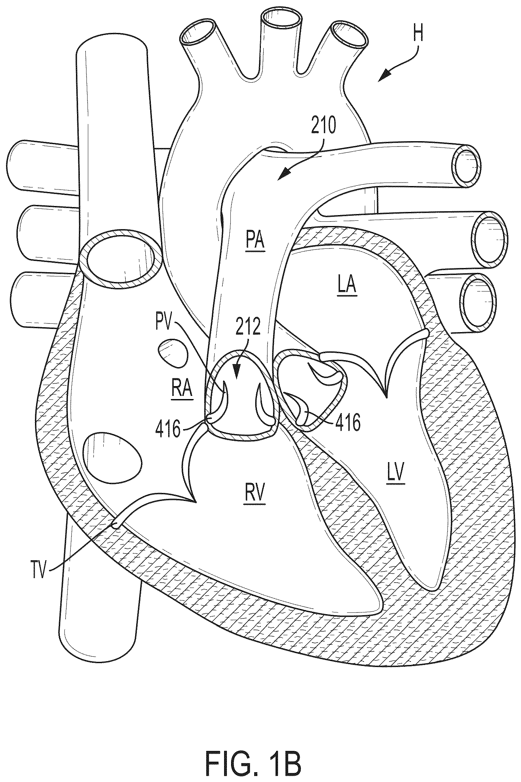

FIGS. 1A and 1B are cutaway views of the human heart H in diastolic and systolic phases, respectively. The right ventricle RV and left ventricle LV are separated from the right atrium RA and left atrium LA, respectively, by the tricuspid valve TV and mitral valve MV; i.e., the atrioventricular valves. Additionally, the aortic valve AV separates the left ventricle LV from the ascending aorta (not identified) and the pulmonary valve PV separates the right ventricle from the pulmonary artery PA. The pulmonary valve PV is disposed at the inlet or start 212 of the pulmonary artery PA. Each of these valves has flexible leaflets extending inward across the respective orifices that come together or "coapt" in the flowstream to form the one-way, fluid-occluding surfaces. The docking stations and valves of the present application are described primarily with respect to the pulmonary valve. Therefore, anatomical structures of the right atrium RA and right ventricle RV will be explained in greater detail. It should be understood that the devices described herein can also be used in other areas, e.g., in the inferior vena cava IVC and/or the superior vena cava SVC as treatment for a regurgitant or otherwise defective tri-cuspid valve, in the aorta (e.g., an enlarged aorta) as treatment for a defective aortic valve AV, in other areas of the heart or vasculature, in grafts, etc.

The right atrium RA receives deoxygenated blood from the venous system through the superior vena cava SVC and the inferior vena cava IVC, the former entering the right atrium from above, and the latter from below. The coronary sinus CS is a collection of veins joined together to form a large vessel that collects deoxygenated blood from the heart muscle (myocardium), and delivers it to the right atrium RA. During the diastolic phase, or diastole, seen in FIG. 1A, the venous blood that collects in the right atrium RA enters the tricuspid valve TV by expansion of the right ventricle RV. In the systolic phase, or systole, seen in FIG. 1B, the right ventricle RV contracts to force the venous blood through the pulmonary valve PV and pulmonary artery PA into the lungs. In one exemplary embodiment, the devices described by the present application are used to replace or supplement the function of a defective pulmonary valve PV. During systole, the leaflets of the tricuspid valve TV close to prevent the venous blood from regurgitating back into the right atrium RA.

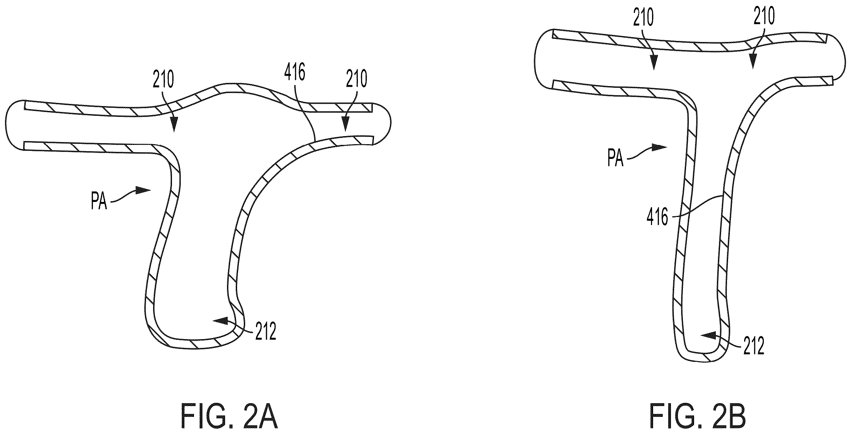

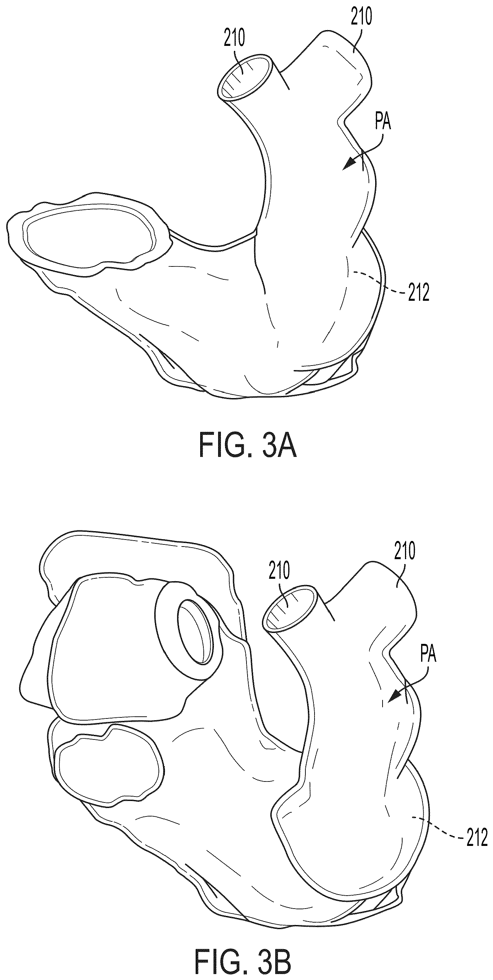

Referring to FIGS. 2A - 2B and 3A - 3B, the shown, non-exhaustive examples illustrate that the pulmonary artery can have a wide variety of different shapes and sizes. For example, as shown in the sectional views of FIGS. 2A and 2B and the perspective views of FIGS. 3A and 3B, the length, diameter, and curvature or contour may vary greatly between pulmonary arteries of different patients. Further, the diameter may vary significantly along the length of an individual pulmonary artery. These differences can be even more significant in pulmonary arteries that suffer from certain conditions and/or have been compromised by previous surgery. For example, the treatment of Tetralogy of Fallot (TOF) or Transposition of the Great Arteries (TGA) often results in larger and more irregularly shaped pulmonary arteries.

Tetralogy of Fallot (TOF) is a cardiac anomaly that refers to a combination of four related heart defects that commonly occur together. The four defects are ventricular septal defect (VSD), overriding aorta (the aortic valve is enlarged and appears to arise from both the left and right ventricles instead of the left ventricle as in normal hearts), pulmonary stenosis (narrowing of the pulmonary valve and outflow tract or area below the valve that creates an obstruction of blood flow from the right ventricle to the pulmonary artery), and right ventricular hypertrophy (thickening of the muscular walls of the right ventricle, which occurs because the right ventricle is pumping at high pressure).

Transposition of the Great Arteries (TGA) refers to an anomaly where the aorta and the pulmonary artery are "transposed" from their normal position so that the aorta arises from the right ventricle and the pulmonary artery from the left ventricle.

Surgical treatment for some conditions involves a longitudinal incision along the pulmonary artery, up to and along one of the pulmonary branches. This incision can eliminate or significantly impair the function of the pulmonary valve. A trans-annular patch is used to cover the incision after the surgery. The trans-annular patch reduces stenotic or constrained conditions of the pulmonary artery PA, associated with other surgeries. However, the impairment or elimination of the pulmonary valve PV can create significant regurgitation and, prior to the present invention, often required later open-heart surgery to replace the pulmonary valve. The trans-annular patch technique can result in pulmonary arteries having a wide degree of variation in size and shape (see, e.g., FIGS. 3A and 3B).

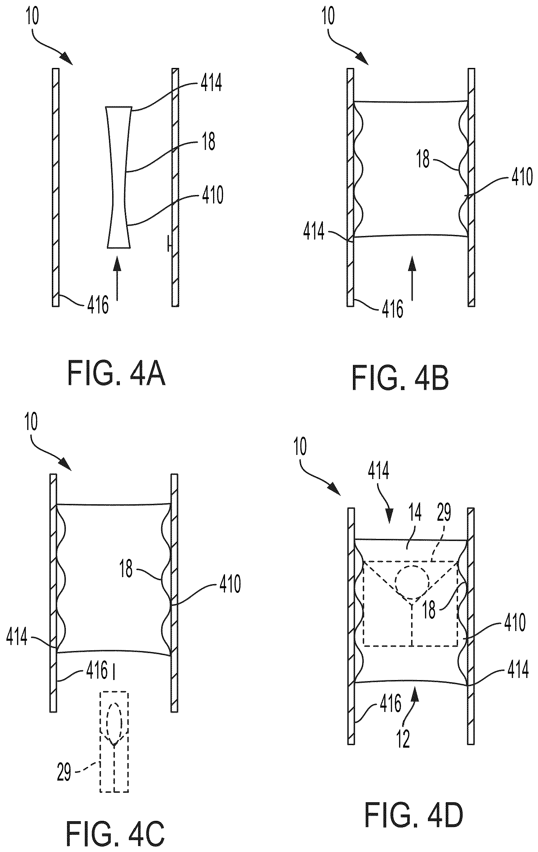

Referring to FIGS. 4A - 4D, in one exemplary embodiment, an expandable docking station 10 includes one or more sealing portions 410, a valve seat 18, and one or more retaining portions 414. The sealing portion(s) 410 provide a seal between the docking station 10 and an interior surface 416 of the circulatory system. The valve seat 18 provides a supporting surface for implanting or deploying a valve 29 in the docking station 10 after the docking station 10 is implanted in the circulatory system. The retaining portions 414 help retain the docking station 10 and the valve 29 at the implantation position or deployment site in the circulatory system. Expandable docking station 10 and valve 29 as described in the various embodiments herein are also representative of a variety of docking stations and/or valves that might be known or developed, e.g., a variety of different types of valves could be substituted for and/or used as valve 29 in the various docking stations.

FIGS. 4A - 4D schematically illustrate an exemplary deployment of the docking station 10 and valve 29 in the circulatory system. Referring to FIG. 4A, the docking station 10 is in a compressed form/configuration and is introduced to a deployment site in the circulatory system. For example, the docking station 10, can be positioned at a deployment site in a pulmonary artery by a catheter (e.g., one or more of the catheters described in co-owned U.S. Patent Application Publication No. 2019/0000615 and U.S. Patent No. 10,363,130, the entire disclosures of both of which are incorporated herein by reference). Referring to FIG. 4B, the docking station 10 is expanded in the circulatory system such that the sealing portion(s) 410 and the retaining portions 414 engage the inside surface 416 of a portion of the circulatory system. Referring to FIG. 4C, after the docking station 10 is deployed, the valve 29 is in a compressed form and is introduced into the valve seat 18 of the docking station. Referring to FIG. 4D, the valve 29 is expanded in the docking station 10, such that the valve engages the valve seat 18. In the examples depicted herein, the docking station 10 is longer than the valve 29. However, in other embodiments the docking station 10 can be the same length or shorter than the length of the valve 29. Similarly, the valve seat 18 can be longer, shorter, or the same length as the length of the valve 29.

Referring to FIG. 4D, the valve 29 has expanded such that the seat 18 of the docking station supports the valve. The docking station 10 allows the valve 29 to operate within the expansion diameter range for which it is designed.

In an exemplary embodiment, the docking station 10 is configured to expand radially outwardly to varying degrees along its length to conform to shape of the inner surface 416. In one exemplary embodiment, the docking station 10 is configured such that the sealing portion(s) 410 and/or the retaining portion(s) engage the inner surface 416, even though the shape of the blood vessel or anatomy of the heart vary significantly along the length of the docking station. The docking station can be made from a very resilient or compliant material to accommodate large variations in the anatomy. For example, the docking station 10 can be made from a highly flexible metal, metal alloy, or polymer. Examples of a metals and metal alloys that can be used include, but are not limited to, nitinol, elgiloy, and stainless steel, but other metals and highly resilient or compliant non-metal materials can be used. For example, the docking station 10 can have a frame or portion of a frame (e.g., a self-expanding frame, retaining portion(s), sealing portion(s), valve seat, etc.) made of these materials, e.g., from shape memory materials, such as nitinol. These materials allow the frame to be compressed to a small size, and then when the compression force is released, the frame will self-expand back to its precompressed diameter. Docking stations described herein can be self-expanding and/or expandable with an inflatable device to cause the docking station to engage an inner surface 416 having a variable shape.

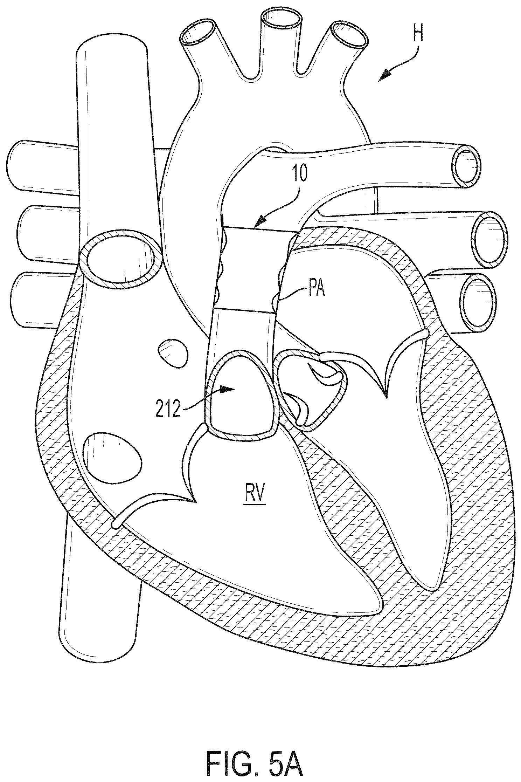

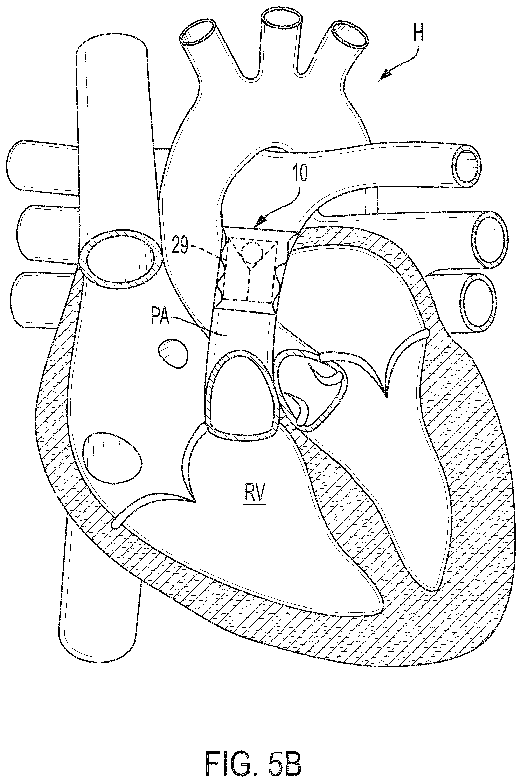

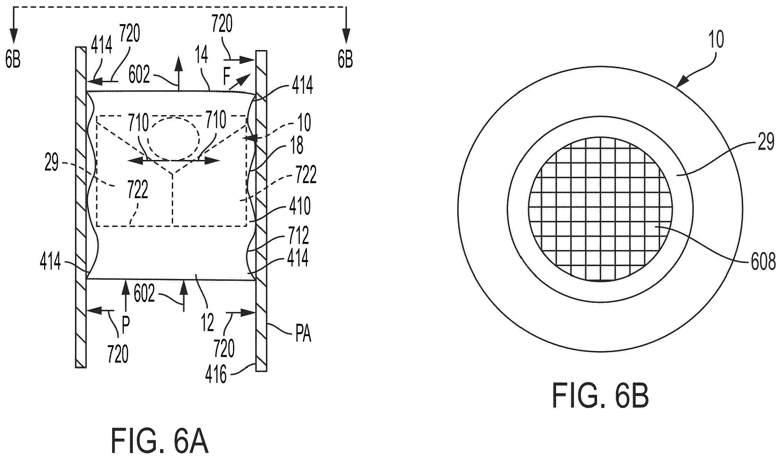

Referring to FIG. 5A, a docking station, e.g., a docking station as described with respect to FIGS. 4A - 4D, is deployed in the pulmonary artery PA of a heart H. FIG. 5B illustrates a valve 29 deployed in the docking station 10 illustrated by FIG. 5A. In FIGS. 6A and 6B, the heart is in the systolic phase. FIG. 6A is an enlarged schematic representation of the docking station 10 and valve 29 in the pulmonary artery PA. When the heart is in the systolic phase, the valve 29 opens. Blood flows from the right ventricle RV and through the pulmonary artery PA, docking station 10, and valve 29 as indicated by arrows 602. FIG. 6B illustrates a blood-filled space 608 that represents the valve 29 being open when the heart is in the systolic phase. FIG. 6B does not show the interface between the docking station 10 and the pulmonary artery to simplify the drawing. The cross-hatching in FIG. 6B illustrates blood flow through the open valve. In an exemplary embodiment, blood is prevented from flowing between the pulmonary artery PA and the docking station 10 by the sealing portion(s) 410 and blood is prevented from flowing between the docking station 10 and the valve 29 by seating of the valve 29 in the seat 18 of the docking station 10. In this example, blood is substantially only flowing or only able to flow through the valve 29 when the heart is in the systolic phase.

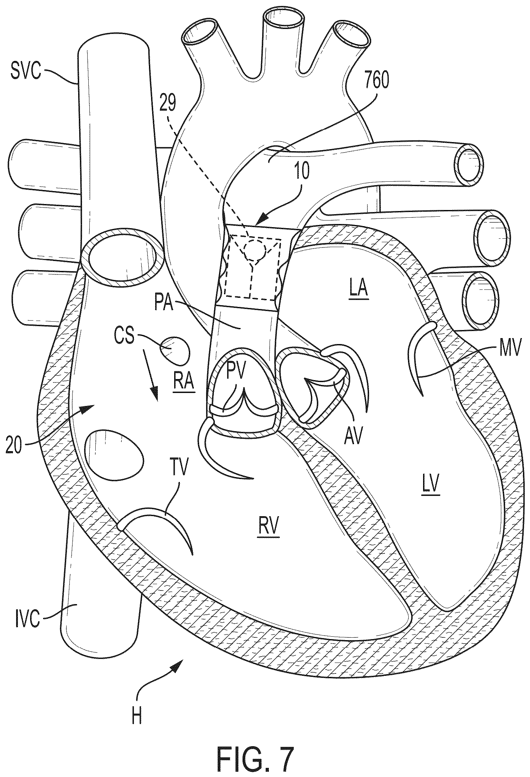

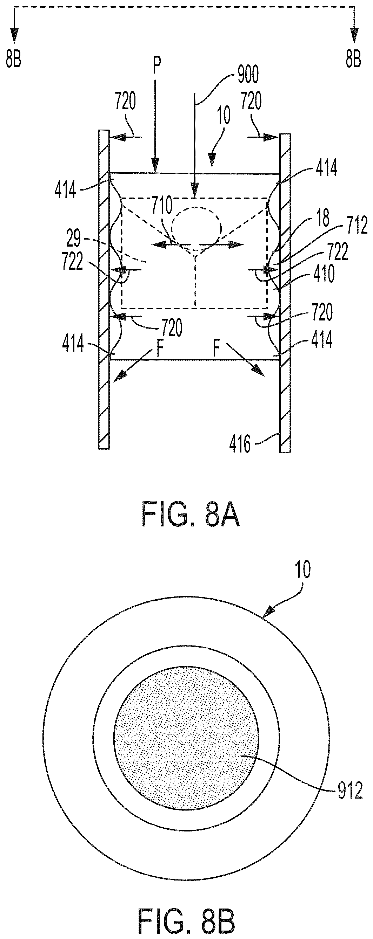

FIG. 7 illustrates the valve 29, docking station 10 and heart H illustrated by FIG. 5B, when the heart is in the diastolic phase. Referring to FIGS. 8A and 8B, when the heart is in the diastolic phase, the valve 29 closes. FIG. 8A is an enlarged schematic representation of the docking station 10 and valve 29 in the pulmonary artery of FIG. 7. Blood flow in the pulmonary artery PA above the valve 29 (i.e., in the pulmonary branch 760) is blocked by the valve 29 being closed and blocking blood flow as indicated by arrow 900. The solid area 912 in FIG. 8B represents the valve 29 being closed when the heart is in the diastolic phase.

In one exemplary embodiment, the docking station 10 acts as an isolator that prevents or substantially prevents radial outward forces of the valve 29 from being transferred to the inner surface 416 of the circulatory system. In one embodiment, the docking station 10 includes a valve seat 18 (which is not expanded radially outwardly or is not substantially expanded radially outward by the radially outward force of the THV or valve 29, i.e., the diameter of the valve seat is not increased or is increased by less than about 4 mm by the force of the THV), and anchoring/retaining portions 414 and sealing portions 410, which impart only relatively small radially outward forces 720, 722 on the inner surface 416 of the circulatory system (as compared to the radially outward force applied to the valve seat 18 by the valve 29).

When no docking station is used, stents and frames of THVs are held in place in the circulatory system by a relatively high radial outward force 710 of the stent or frame 712 of the THV acting directly on the inside surface 416 of the circulatory system. If a docking station is used, as in the example illustrated by FIG. 6A, the stent or frame 712 of the valve 29 expands radially outward or is expanded radially outward to impart the high force 710 on the valve seat 18 of the docking station 10. This high radially outward force 710 secures the valve 29 to the valve seat 18 of the docking station 10. However, since the valve seat 18 is not expanded or is not substantially expanded by the force 710, the force 710 is isolated from the circulatory system, rather than being used to secure the docking station in the circulatory system.

In an exemplary embodiment, the radially outward force 722 of the sealing portions 410 to the inside surface 416 is substantially smaller than the radially outward force 710 applied by the valve 29 to the valve seat 18. For example, the radially outward sealing force 722 can be less than ½ the radially outward force 710 applied by the valve, less than 1/3 the radially outward force 710 applied by the valve, less than 1/4 the radially outward force 710 applied by the valve, less than 1/8, or even less than 1/10 the radially outward force 710 applied by the valve. In one exemplary embodiment, the radially outward force 722 of the sealing portions 410 is selected to provide a seal between the inner surface 416 and the sealing portion 410, but is not sufficient by itself to retain the position of the valve 29 and docking station 10 in the circulatory system.

In an exemplary embodiment, the radially outward force 720 of the anchoring/retaining portions 414 to the inside surface 416 is substantially smaller than the radially outward force 710 applied by the valve 29 to the valve seat 18. For example, the radially outward sealing force 720 can be less than ½ the radially outward force 710 applied by the valve, less than 1/3 the radially outward force 710 applied by the valve, less than 1/4 the radially outward force 710 applied by the valve, less than 1/8, or even less than 1/10 the radially outward force 710 applied by the valve.

In one exemplary embodiment, the radially outward force 720 of the retaining portions 414 is not sufficient by itself to retain the position of the valve 29 and docking station 10 in the circulatory system. Rather, the pressure of the blood 608 is used to enhance the retention of the retaining portions 414 to the inside surface 416. Referring again to FIG. 6A, when the heart is in the systolic phase, the valve 29 is open and blood flows through the valve as indicated by arrows 602. Since the valve 29 is open and blood flows through the valve 29, the pressure P applied to the docking station 10 and valve 29 by the blood is low as indicated by the small arrow P in FIG. 6A. Even though small, the pressure P forces the docking station and its upper retaining portions 414 against the surface 416 generally in the direction indicated by arrow F. This radially outward directed blood flow assisted force F applied by the retaining portions 414 to the surface 416 impedes the docking station 10 and valve 29 from moving in the direction 602 of blood flow in the systolic phase of the heart H.

Referring to FIG. 8A, when the heart is in the diastolic phase, the valve 29 is closed and blood flow is blocked as indicated by arrow 900. Since the valve 29 is closed and the valve 29 and docking station 10 block the flow of blood, the pressure P applied to the docking station 10 and valve 29 by the blood is high as indicated by the large arrow P in FIG. 8A. This large pressure P forces the lower retaining portions 414 against the surface 416 generally in the direction indicated by the large arrows F. This radially outward directed blood flow assisted force F applied by the retaining portions 414 to the surface 416 prevents the docking station 10 and valve 29 from moving in the direction indicated by arrow 900.

Since the force applied by the upper and lower retaining portions 414 is determined by amount of pressure applied to the valve 29 and docking station 10 by the blood, the force applied to the surface 416 is automatically proportioned. That is, the upper retaining portions are less forcefully pressed against the surface 416 when the heart is in the systolic phase than the lower retaining portions are pressed against the surface 416 when the heart is in the diastolic phase. This is because the pressure against the open valve 29 and docking station 10 in the systolic phase is less than the pressure against the closed valve and docking station in the diastolic phase.

The valve seat 18, sealing portions 410, and retaining portions 414 can take a wide variety of different forms. For example, the valve seat 18 can be any structure that is not expanded radially outwardly or is not substantially expanded radially outward (e.g., expanding 1-4 mm larger when the valve is deployed in the valve seat) by the radially outward force of the THV, and can, for example, be restricted in expansion by a band (e.g., suture, rigid ring) secured to or integral with the narrower waist portion of the docking station 10. In one such example, the expanded frame 1500 may define a valve seat having a diameter of about 27 mm (e.g., about 27.1 mm) that expands to a diameter of about 29 mm to accommodate a valve 29 having a deployed, expanded diameter of about 29 mm.

The sealing portion 410 may, for example, be defined by a wider portion of the docking station 10 (i.e., relative to the narrower waist portion), provided with a sealing material, such as, for example, a fabric, foam, or biocompatible tissue attached to or integral with the docking station.

The retaining portions 414 may, for example, be defined by flared ends of a docking station frame, which are angled for anchoring engagement with the inner surface 416 of the circulatory system.

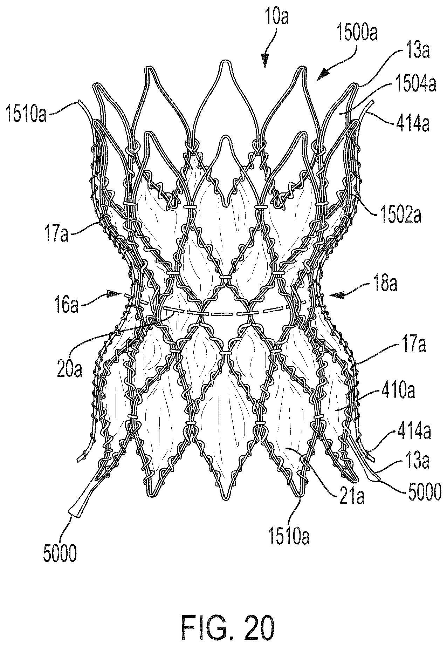

Co-owned U.S. Patent No. 10,363,130 (the "'130 Patent"), the entire disclosure of which is incorporated herein by reference, describes and shows several exemplary docking stations comprising expandable frames formed form a lattice of metal strut portions. FIG. 20 illustrates one such exemplary docking station 10a from the '130 Patent, including a generally hourglass shaped frame 1500a formed by a lattice of strut portions 1502a having a central portion constricted by a band 20a to provide a narrow waist portion 16a defining the valve seat 18a, convex contoured medial portions 17a having an attached impermeable material 21a to define tissue engaging sealing portions 410a, and outwardly flared end portions 13a, with the apices 1510a of the endmost cells 1504a defining outwardly flared retaining portions 414a angled to anchor against the inner surface of the circulatory system.

According to an aspect of the present disclosure, a docking station frame defining a central waist portion, medial sealing portions, and outer retaining portions may be formed with a substantially continuous curved longitudinal profile, providing a substantially smooth transition from the central waist portion to the maximum frame diameter at the outer ends. As used herein, "substantially continuous curved" profiles may include (a) profiles having a continuous radius of curvature between the proximal and distal ends and across the waist portion and (b) profiles having a radius of curvature that increases from each of the proximal and distal ends toward the central waist portion, to provide a flatter waist portion and more sharply angled end portions. In some embodiments, the radius of curvature may transition, either gradually or directly from each of the proximal and distal ends toward a substantially cylindrical (i.e., having a radius of curvature that is infinite or approaching infinite) central waist portion.

In some applications, these types of substantially continuous curved longitudinal profile, by minimizing discrete or localized changes in the profile contour angle, may reduce the localized stress concentrations on the frame, for example, to improve fracture resistance of the docking station frame strut portions. In one such embodiment, a large radius of the substantially continuous outward curved or concave longitudinal profile may provide a longer waist region (and a longer resulting valve seat), and/or a reduced angle at the apices of the frame, for example, to limit or prevent perforation or penetration of the engaged tissue.

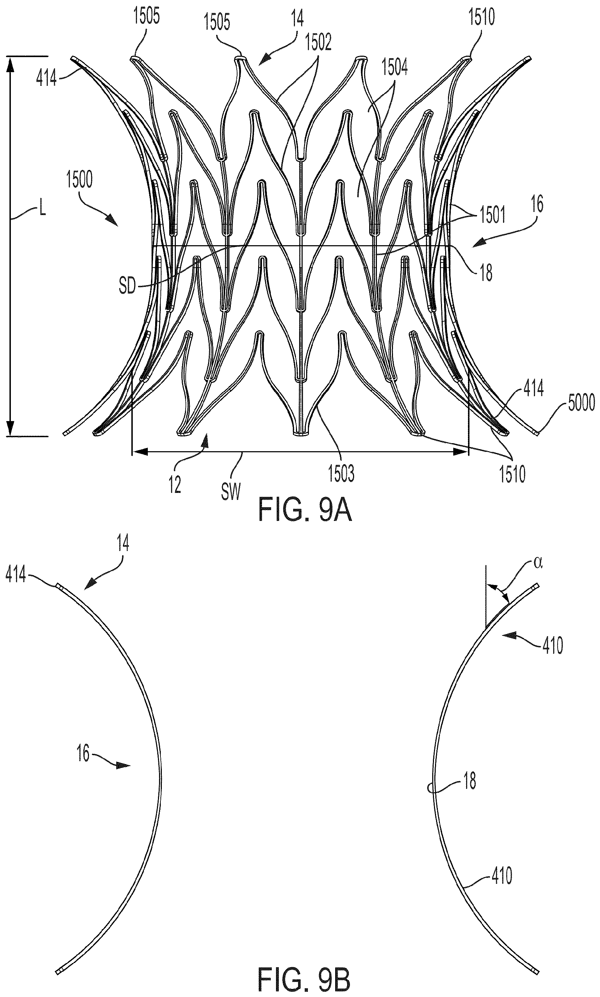

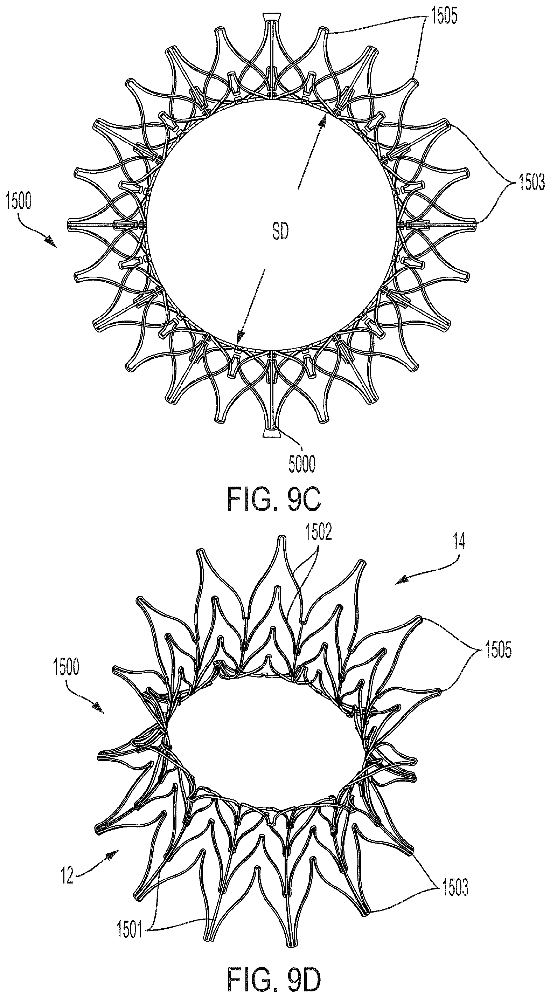

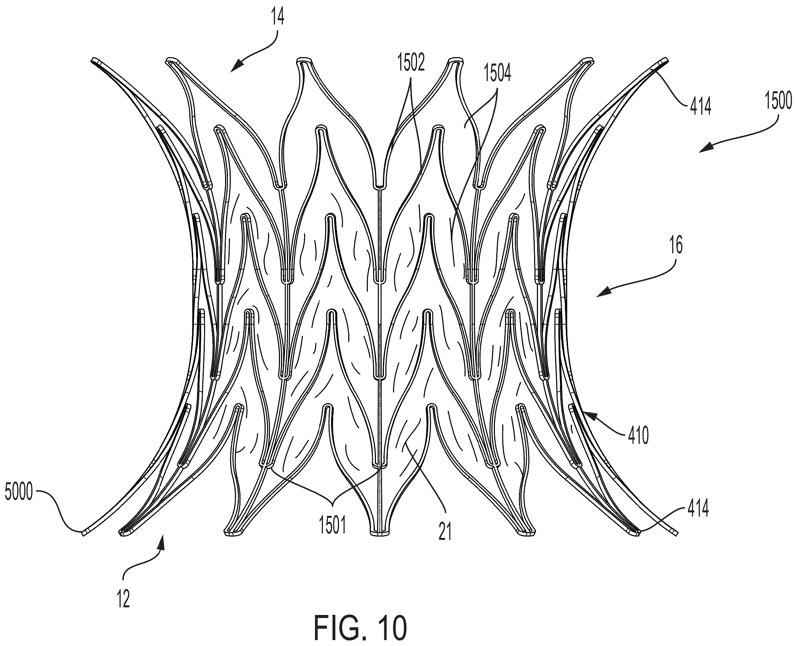

FIGS. 9A - 9D illustrate an exemplary embodiment of a frame 1500 or body of a docking station 10. The frame 1500 or body can take a wide variety of different forms and FIGS. 9A - 9D illustrate just one of the many possible configurations. In the examples illustrated by FIGS. 9A - 9D and 10, the docking station 10 has a relatively wider proximal inflow end 12 and distal outflow end 14, and a relatively narrower portion 16 that forms the valve seat 18 in between the proximal (e.g., inflow) and distal (e.g., outflow) ends 12, 14. In the example illustrated by FIGS. 9A - 9D, the frame 1500 of the docking station 10 is preferably a wide stent comprised of a lattice of metal strut portions 1501, 1502 forming enclosed spaces and defining cells 1504. In the example of FIGS. 9A - 9D, the frame 1500 has a generally concave longitudinal profile around its circumference, which defines a relatively narrower (compared to the outer ends), cylindrical or shallow concave central portion 16 forming the valve seat 18 when covered by an impermeable material, in between the proximal and distal ends 12, 14. As described below, in use, the valve 29 is aligned with, and expands in, the narrower central portion 16, which forms the valve seat 18.

FIGS. 9A - 9D illustrate the frame 1500 in its unconstrained, expanded condition. In this exemplary embodiment, the retaining portions 414 comprise ends or apices 1510 (formed by junctions 1503, 1505, described below) of the metal strut portions 1502 at the proximal (e.g., inflow) and distal (e.g., outflow) ends 12, 14. The sealing portions 410 are disposed medially between the retaining portions 414 and the narrower central portion 16. In the unconstrained condition, the retaining portions 414 extend generally radially and axially outward (e.g., at an angle of between about 20° and about 60°, or between about 30° and about 45°, or about 45° with respect to the central axis) and are radially outward of the sealing portions 410.

The docking station can be made from a very resilient or compliant material to accommodate large variations in the anatomy. For example, the docking station can be made from a highly flexible metal, metal alloy, polymer, or an open cell foam. An example of a highly resilient metal is nitinol, but other metals and highly resilient or compliant non-metal materials can be used. The docking station frame 1500 can be self-expanding, manually expandable (e.g., expandable via balloon), or mechanically expandable. A self-expanding docking station frame 1500 can be made of a shape memory material such as, for example, nitinol. In an exemplary embodiment, the unconstrained retaining portions 414 extend to an outer diameter of about 41 mm, and are flexible enough to engage and be retained in circulatory system vessels having a diameter between about 27 mm and about 38 mm.

In the illustrated embodiment, the docking station frame 1500 is formed with a substantially continuous curved longitudinal profile, providing a substantially smooth transition from the central waist portion 16 to the maximum frame diameter at the outer ends 12, 14. In some applications, this type of substantially continuous curved longitudinal profile, by minimizing discrete or localized changes in the profile contour angle, may reduce the localized stress concentrations on the frame 1500, for example, to improve fracture resistance of the docking station frame strut portions 1501, 1502. In one such embodiment, the outward curved or concave longitudinal profile may provide a longer central waist portion 16, and a longer resulting valve seat 18. For example, the cylindrical or shallow concave central waist portion 16 may provide an effective valve seat length of at least about 8 mm, or between about 9 mm and about 10 mm at a deployed valve diameter of about 29 mm. The resulting large outward curved or concave length of the longitudinal profile may additionally or alternatively provide a reduced angle at the apices 1510 of the frame 1500, for example, to limit or prevent perforation or penetration of the engaged tissue by the retaining portions 414. For example, the apices 1510 of the frame 1500, when in an expanded, unconstrained condition, may extend at an angle α between about 20° and about 60°, or between about 30° and about 45°, or about 45° with respect to the longitudinal axis of the frame. In an exemplary embodiment, the narrow waist portion 16 has a cylindrical length of about 3 mm to about 6 mm, or about 4 mm to about 5 mm, or about 4 mm, which transitions (either gradually or directly) to a radius of curvature of between about 14 mm and about 20 mm, or about 19 mm, at the outer ends 12, 14.

The frame 1500 of the docking station 10 can be sized, shaped, and/or otherwise configured to fit pulmonary arteries of varying sizes, shapes, diameters, and geometries. The frame 1500 of the docking station 10 can have any number of strut portions 1502, any number of cells 1504, or any number of apices 1510, or the strut portions 1501, 1502 or the cells 1504 can have any shape to fit pulmonary arteries of varying sizes, shapes, and geometries. The strut portions 1501, 1502 can have any size, shape, thickness, or configuration to retain the valve 29 in the pulmonary artery PA. Additionally, the proximal end 12 of the frame 1500 can have a different size, shape, and/or configuration from the distal end 14 of the frame 1500.

As shown in FIG. 9A, the frame 1500 can have a height H extending from the proximal end 12 to the distal end 14 of the frame and a seat diameter SD which is the diameter of the valve seat 18. The frame 1500 can also have a seal width SW which is the width of the sealing portion 410 at the point between the proximal end 12 and the valve seat 18 where the docking station 10 seals with the pulmonary artery.

The frame 1500 of the docking station 10 can have different numbers of rows of cells 1504. The number and configuration of rows can be determined to provide a better securement, fit, or apposition of the docking station 10 in the pulmonary artery PA. For example, the docking station 10 can include more rows of cells 1504 for longer pulmonary arteries PA or where more radial force is beneficial.

The frame 1500 of the docking station 10 can be configured for wide pulmonary arteries PA. For example, the frame 1500 of the docking station 10 can be configured for pulmonary arteries PA that are short and wide. In one such example, the frame 1500 can have a height H between 32 mm and 45 mm, such as between about 35 mm and about 40 mm, such as about 38 mm. The exemplary frame 1500 can have a seat diameter SD between 23 mm and 32 mm, such as between about 26 mm and 29 mm, such as about 27 mm. The exemplary frame can have end diameters of between 39 mm and 50 mm, such as about 41 mm. The frame 1500 can have a seal width SW between 36 mm and 46 mm, such as between about 38 mm and 44 mm, such as about 41 mm.

The frame 1500 of the docking station 10 can also be configured to fit a longer and/or wider pulmonary artery. For example, the frame 1500 of the docking station 10 can be longer and wider. In one such example, the frame 1500 of the docking station 10 can have a height H between 43 mm and 53 mm, such as between about 45 mm and about 51 mm, such as about 48 mm. The frame 1500 can have a seat diameter SD between 24 mm and 31 mm, such as between about 26 mm and about 29 mm, such as about 27 mm. The frame 1500 can have a seal width SW between 44 mm and 54 mm, such as between about 46 mm and about 52 mm, such as about 48 mm or about 50 mm.

While the frame 1500 has been described as having herringbone shaped cells 1504, the frame can also have alternative configurations or geometries such that the frame 1500 does not have herringbone-shaped cells 1504 or not all the cells 1504 are herringbone-shaped.

FIG. 10 illustrates a docking station 10 including the frame 1500 with impermeable material 21 attached to the frame 1500 to form the valve seat 18 and sealing portions 410 of the docking station 10, as described in greater detail below. In some embodiments, the frame 1500 may be provided without a restricting band in the waist portion, instead relying on the inherent stiffness and flexibility limits of the inner radial portion of the longitudinal frame profile. In other embodiments, the frame may be provided with a band configured to stiffen and constrict the narrow waist portion 16, as disclosed in the above incorporated '130 Patent, and shown in FIG. 20 herein. In one such embodiment, a suture used to attach the impermeable material 21 to the waist portion 16 of the frame 1500 may additionally function as a waist stiffening/reinforcing band.

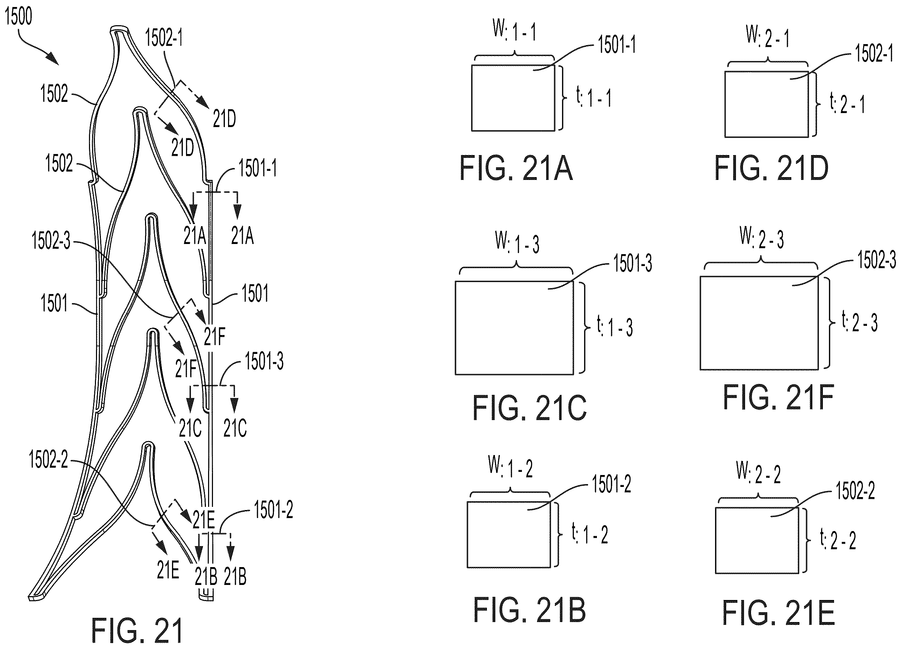

According to another exemplary aspect of the present disclosure, the frame struts 1501, 1502 may be configured to vary in circumferential width and/or radial thickness to provide increased or decreased flexibility and/or increased or decreased radial forces for desired engagement between the retaining portions 414 and the internal surface IS and between the valve seat 18 and the valve. In one such embodiment, as shown in Figures 21 and 21A - 21F, the struts 1501, 1502 of an expandable frame 1500 may have distal first end portions 1501-1, 1502-1 (defining the distal or first end cells 1504-1) having a first cross-sectional area, second end or proximal portions 1501-2, 1502-2 (defining the proximal or second end cells 1504-2) having a second cross-sectional area, and central portions 1501-3, 1502-3 (defining the central cells 1504-3) having a third cross-sectional area. In the illustrated example, the third cross-sectional area of the central strut portions 1501-3, 1502-3 is greater than the first and second cross-sectional areas of the first and second end strut portions 1501-1, 1502-1, 1501-2, 1502-2 (which, may, but need not, be substantially the same). The greater cross-sectional area of the central strut portions 1501-3, 1502-3 may provide for reduced flexibility (e.g., to isolate the valve seating waist portion from the deployment site) and increased radial force (e.g., to securely retain the seated valve) at the central portion of the frame 1500, while the smaller cross-sectional area of the first and second end strut portions 1501-1, 1502-1, 1501-2, 1502-2 may provide for increased flexibility (e.g., to conform to the internal surface contours at the deployment site) and reduced but sufficient radial force (e.g., to minimize or prevent tissue damage by the retaining portions 414) at the proximal and distal end portions, for example, to maintain a chronic outward force (COF) of at least about 25 Newtons for sufficient anchoring of the frame 1500 at the deployment site while maintaining flexibility for compliance with the contours of the tissue at the deployment site.

As shown, the central strut portions 1501-3, 1502-3 may have a greater radial thickness t₁₋₃, t₂₋₃ than a thickness t₁₋₁, t₂₋₁ and/or t₁₋₂, t₂₋₂ of the distal and/or proximal strut portions 1501-1, 1502-1, 1501-2, 1502-2 and/or a greater circumferential width w₁₋₃, w₂₋₃ than a width w₁₋₁, w₂₋₁ and/or w₁₋₂, w₂₋₂ of the distal and/or proximal strut portions 1501-1, 1502-1, 1501-2, 1502-2. For example, the circumferential width w₁₋₃, w₂₋₃ can be 110% to 300% of the width w₁₋₁, w₂₋₁ and/or w₁₋₂, w₂₋₂, such as 110% to 130% of the width w₁₋₁, w₂₋₁ and/or w₁₋₂, w₂₋₂, such as about 115% to about 120% of the width w₁₋₁, w₂₋₁ and/or w₁₋₂, w₂₋₂. In an exemplary embodiment, the central strut portions 1501-3, 1502-3 may have a circumferential width w₁₋₃, w₂₋₃ of about 0.29 mm, and the distal and/or proximal strut portions 1501-1, 1502-1, 1501-2, 1502-2 may have a circumferential width w₁₋₁, w₂₋₁ and/or w₁₋₂, w₂₋₂ of about 0.24 mm. As another example, the central strut portions 1501-3, 1502-3 may have a circumferential width w₁₋₃, w₂₋₃ of about 0.39 mm, and the distal and/or proximal strut portions 1501-1, 1502-1, 1501-2, 1502-2 may have a circumferential width w₁₋₁, w₂₋₁ and/or w₁₋₂, w₂₋₂ of about 0.34 mm. Likewise, the radial thickness t₃₋₁, t₃₋₂ can be 110% to 300% of the thickness t₁₋₁, t₂₋₁ and/or t₁₋₂, t₂₋₂, such as 110% to 130% of the thickness t₁₋₁, t₂₋₁ and/or t₁₋₂, t₂₋₂, such as 115% to 120% of the thickness t₁₋₁, t₂₋₁ and/or t₁₋₂, t₂₋₂. In other embodiments, the circumferential width of the strut portions may vary, as described above, with the radial thickness of the strut portions being substantially constant, for example, to facilitate formation of the frame lattice from a tube having a uniform wall thickness.

The unexpandable or substantially unexpandable valve seat may prevent the radially outward force of the valve from being transferred to the inside surface of the circulatory system. However in another exemplary embodiment, the waist/valve seat of the deployed docking station can optionally expand slightly in an elastic fashion when the valve is deployed against it. This optional elastic expansion of the waist/valve seat can put pressure on the valve to help hold the valve in place within the docking station.

The strut portions of the docking station frame may be configured to form a variety of lattice patterns and cell shapes, such as, for example, the substantially diamond shaped cell configurations of the embodiment of FIG. 20 and embodiments disclosed in the above incorporated '130 Patent. In the illustrated example of FIGS. 9A - 9D and 10, the docking station frame 1500 has a lattice frame arrangement including axially spaced rows of undulating circumferential strut portions or rungs 1502, arranged in a parallel zigzag pattern defining proximal and distal junctions 1503, 1505, and circumferentially spaced longitudinal strut portions or splines 1501, extending longitudinally along the proximal junctions 1503 to define rows of substantially herringbone shaped cells 1504. The proximal and distal junctions 1503, 1505 may be configured to function as hinges to bias the frame 1500 radially outward into engagement with the surrounding tissue. The distal junctions 1505 of the undulating circumferential strut portions 1502 are unattached or "free" from the longitudinal strut portions 1501, allowing for outward flexing of these distal junctions to facilitate vasculature tissue engagement (e.g., at the distal retaining portion 414 and the sealing portion(s) 410) when implanted, thereby resisting forward distal movement of the implanted frame 1500, as discussed in greater detail below. The vertical strut attachment to the proximal junctions 1503 limits outward flexing of the cells at the proximal junctions to facilitate retraction of the frame 1500 to a crimped or compressed size (e.g., for retrieval into the catheter), for example, to a diameter of less than about 7 mm or about 5.4 mm, with a limited radial resistive force (e.g., less than 60 N) at the fully crimped diameter. The retaining portions 414 at each end may have substantially the same maximum diameter (e.g., between about 42 mm and about 45 mm, or about 44 mm). In other embodiments, due to the unattached condition of the distal junctions 1505, the distal or outflow end 14 of the frame 1500 may have a slightly larger diameter in the expanded, unconstrained condition.

The frame 1500 may include any suitable number of rows of cells 1504 (e.g., at least three, or at least four, or at least five), and any suitable number of cells per row (e.g., at least ten, or at least twelve). In the illustrated example, the frame 1500 includes five undulating circumferential strut portions 1502 defining four rows of cells 1504, and twelve longitudinal strut portions 1501 defining twelve cells 1504 per row. By using the herringbone shaped cell arrangement, a greater number of circumferential strut portions 1502 (and resulting rows of cells) may be provided over the length of the frame (for example, compared to the diamond-shaped cell arrangement), for example, to increase the strength of the frame 1500, to provide high device retention, crush resistance, and/or lower radial outward force (e.g., to maintain device integrity while minimizing tissue damage). Additionally or alternatively, the frame 1500 may be provided in a shorter length (e.g., about 35 - 40 mm in length for a docking station sized for implantation in a pulmonary artery) while still providing sufficient frame strength. The greater number of longitudinal strut portions 1501 (and resulting cells per row) may, for example, provide for increased flexibility in conforming the shape of the frame 1500 to the shape of the deployment site in the circulatory system.

The valve 29 can be delivered to the site of the docking station via conventional means, such as by balloon or mechanical expansion or by self-expansion. When the valve 29 is expanded, it nests in the valve seat of the docking station 10. In one embodiment, the waist portion 16 is slightly elastic and exerts an elastic force against the valve 29, to help hold the valve in place.

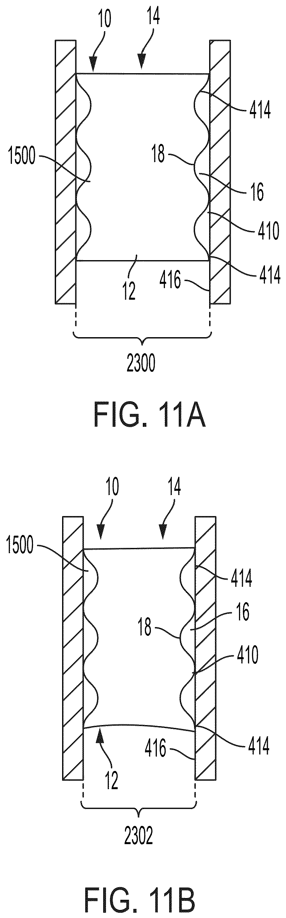

FIGS. 11A and 11B illustrate that the docking station 10 can be used to adapt a variety of different sizes of circulatory system anatomies for implantation of a valve 29 having a consistent size. In the example of FIGS. 11A and 11B, the same size docking station 10 is deployed in two different sized vessels 2300, 2302, such as two differently sized pulmonary arteries PA. In the example, the vessel 2300 illustrated by FIG. 11A has a larger effective diameter than the vessel 2302 illustrated by FIG. 11B. (Note that in this patent application the size of the anatomy of the circulatory system is referred to by the term "diameter" or "effective diameter." The anatomy of the circulatory system is often not circular. The terms "diameter" and "effective diameter" herein refers to the diameter of a circle or disc that could be deformed to fit within the non-circular anatomy.) In the example illustrated by FIGS. 11A and 11B, the sealing portion 410 and the retaining portions 414 conform to contact each vessel 2300, 2302. However, the valve seat 18 remains the same size, even though the sealing portion 410 and the retaining portions 414 are compressed. In this manner, the docking station 10 adapts a wide variety of different anatomical sizes for implantation of a standard or single sized valve. For example, the docking station can conform to vessel diameters of between 25 mm and 40 mm, such as between about 27 mm and 38 mm and provide a constant or substantially constant diameter valve seat of 24 mm to 30 mm, such as between about 27 mm to 28 mm, and a wide range of vessel length, including, for example, pulmonary arteries having a length up to about 45 mm. However, the valve seat 18 can be adapted for applications where the vessel diameter is larger or smaller than 25 mm to 40 mm and provide valve seats that are larger or smaller than 24 mm to 30 mm.

Referring to FIGS. 11A and 11B, the valve seat 18 may be configured to maintain a constant or substantially constant diameter, even as the proximal and distal ends of the docking station expand to respective diameters necessary to engage with the inside surface 416. The diameter of the pulmonary artery PA can vary considerably from patient to patient, but the valve seat 18 in the deployed configuration consistently has a diameter that is within an acceptable range for the valve 29 (e.g., between about 27 mm and about 29 mm).

In an exemplary embodiment, the docking station 10 illustrated by FIG. 10 also includes anchoring/retaining portions 414 that apply radially outward forces that are substantially smaller than the radially outward force applied by the valve 29 to the valve seat 18. For example, for the smallest vessel to be adapted by the docking station 10 for valve implantation, the radially outward sealing force 720 can be less than ½ the radially outward force 710 applied by the valve, less than 1/3 the radially outward force 710 applied by the valve, less than 1/4 the radially outward force 710 applied by the valve, less than 1/8, or even less than 1/10 the radially outward force 710 applied by the valve. In one embodiment, the radially outward force 720 of the anchoring/retaining portions 414 is not sufficient by itself to retain the position of the valve 29 and docking station 10 in the circulatory system. In one embodiment, the radially outward force 720 is sufficient to retain the position of the valve 29 and docking station 10 in the circulatory system.

In one exemplary embodiment, the docking station 10 frame 1500 is made from an elastic or superelastic material or metal. One such metal is nitinol. When the frame 1500 of the docking station 10 is made from a lattice of metal strut portions, the body can have the characteristics of a spring. As the frame 1500 of the docking station 10 is compressed, like a spring, the radially outward force applied by the docking station increases. In one exemplary embodiment the relationship of the radially outward force of the docking station frame 1500 to the expanded diameter of the docking station is non-linear, though it can also be linear.

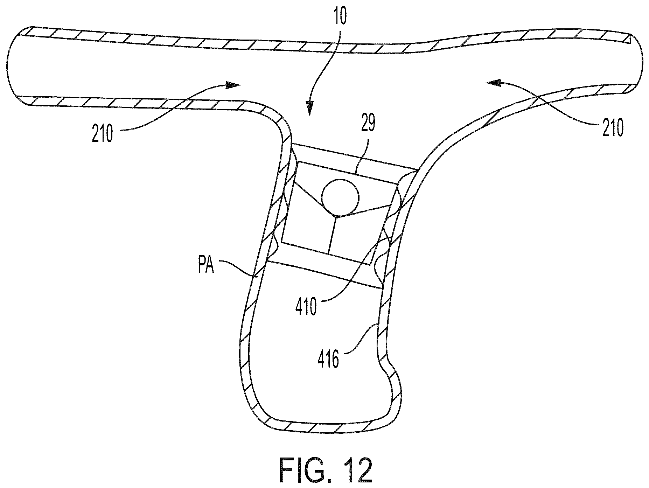

FIG. 12 illustrates the profile of the docking station 10 implanted in the pulmonary artery PA with a schematically illustrated valve 29 installed or deployed in the docking station 10. As mentioned with respect to FIGS. 2A and 2B and 3A and 3B, the shape of the pulmonary artery may vary significantly along its length. In one exemplary embodiment, the docking station 10 is configured to conform to the varying shape of the pulmonary artery PA. The docking station 10 is illustrated as being positioned below the pulmonary artery bifurcation or branch. However, often the docking station 10 will be positioned such that the distal or outflow end 14 extends into the pulmonary artery bifurcation 210. When it is contemplated that the docking station 10 will extend into the pulmonary artery bifurcation, the docking station 10 can have a blood permeable portion 1400.

In embodiments utilizing a docking station frame having a substantially continuous curved longitudinal profile, such as the docking station frame 1500 of FIGS. 9A - 9D and 10, deployment of the docking station 10 at an implantation site in the circulatory system (e.g., the pulmonary artery) causes the radially flared retaining portions 414 to engage the inner surface of the vessel. In some applications, an interference fit between the expanded docking station frame 1500 and the inner surface 416 causes the ends of the docking station frame to flex radially inward, exerting a radially outward force at the retaining portions 414 and potentially creating radial gaps between the docking station frame 1500 and the inner surface 416 at locations between the retaining portions 414 and the sealing portions 410. Additionally, the substantially continuous curved longitudinal profile of the docking station frame 1500 may result in a radial gap between the waist portion 16 and the inner surface 416. In some applications, for example, due to the non-cylindrical shape of the pulmonary artery or other deployment site, one or more of the retaining portion apices may not contact the inner surface 416, for example, during the systolic phase when lower blood pressure provides less pressure against the retaining portions 414.

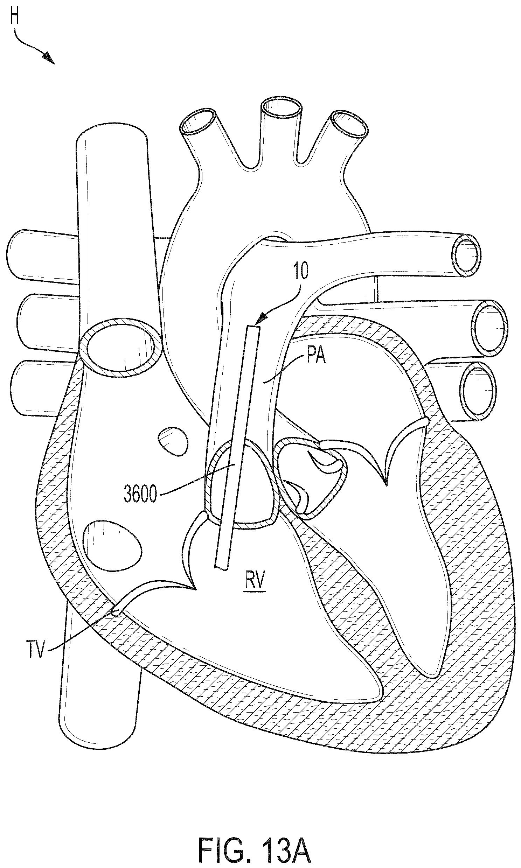

Methods of treating a patient (e.g., methods of treating heart valve dysfunction/regurgitation/etc.), or carrying out a simulation of treating a patient, can include a variety of steps, including steps associated with introducing and deploying a docking station in a desired location/treatment area and introducing and deploying a valve in the docking station. For example, FIG. 13A illustrates the docking station illustrated by FIG. 10 being deployed by a catheter 3600 including a tube retaining the docking station prior to deployment. The docking station 10 can be positioned and deployed in a wide variety of different ways. Access can be gained through the femoral vein or access can be percutaneous. Generally, any vascular path that leads to the pulmonary artery can be used. In one exemplary embodiment, a guidewire followed by a catheter 3600 is advanced to the pulmonary artery PA by way of the femoral vein, inferior vena cava, tricuspid valve and right ventricle RV. The docking station 10 can be placed in the right ventricular outflow tract/pulmonary artery PA to create an artificial conduit and landing zone for a valve (e.g., a transcatheter heart valve) 29.

As shown, the proximal end 12 of the frame 1500 may include extensions 5000, which may extend axially and radially beyond the proximal strut apices to provide limited retaining engagement of the frame 1500 by a catheter during deployment of the frame. Aspects of this feature are described in greater detail in the above incorporated '130 Patent.

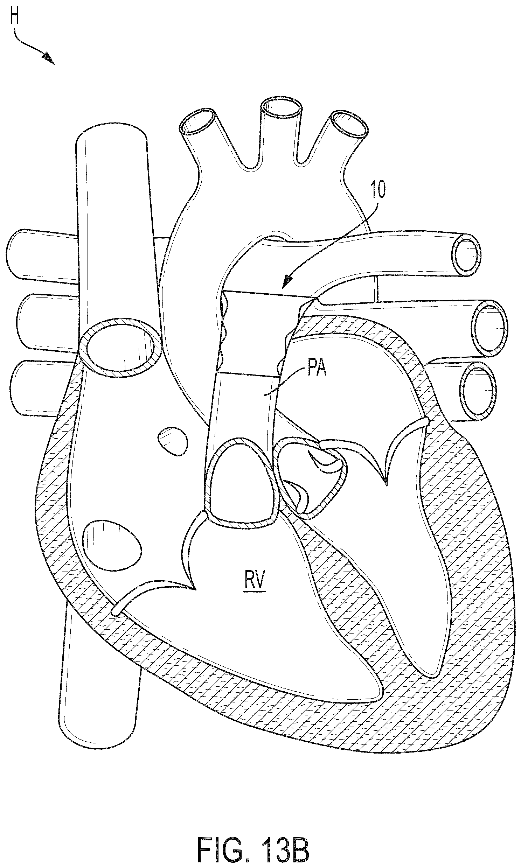

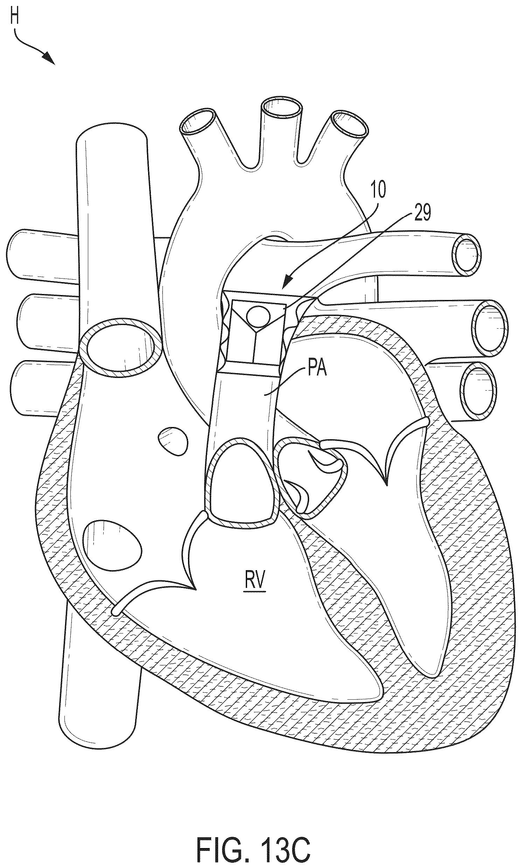

Referring to FIG. 13B, the docking station illustrated by FIG. 10 is deployed in the pulmonary artery (PA) of a heart H. FIG. 13C illustrates a schematically shown valve 29 deployed in the docking station 10. In an exemplary embodiment, the valve 29 may be a SAPIEN 3 THV provided by Edwards Lifesciences; however, a variety of other valves can also be used. In FIGS. 13A - 13C, the heart is in the systolic phase. When the heart is in the systolic phase, the valve (e.g., SAPIEN 3 valve) is open. Blood flows from the right ventricle RV and through the pulmonary artery PA, docking station 10, and valve 29. In an exemplary embodiment, blood is prevented from flowing between the pulmonary artery PA and the docking station 10 by the sealing portion 410 and blood is prevented from flowing between the docking station 10 and the valve by seating of the valve in the seat 18 of the docking station 10. In this example, blood is substantially only or only able to flow through or past the valve when the heart is in the systolic phase.

When the heart is in the diastolic phase, the valve 29 closes. Blood flow in the pulmonary artery PA above the valve 29 (i.e., in the pulmonary branch 210) is blocked by the valve 29 being closed and blocking blood flow, and by fluid tight seals between the sealing portion 410 and the inner surface, and between the valve seat 18 and the valve.

The valve 29 used with the docking station 10 can take a wide variety of different forms. In one exemplary embodiment, the valve 29 is configured to be implanted via a catheter in the heart H. For example, the valve 29 can be expandable and collapsible to facilitate transcatheter application in a heart. However, in other embodiments, the valve 29 can be configured for surgical application. Similarly, the docking stations described herein can be placed using transcatheter application/placement or surgical application/placement.

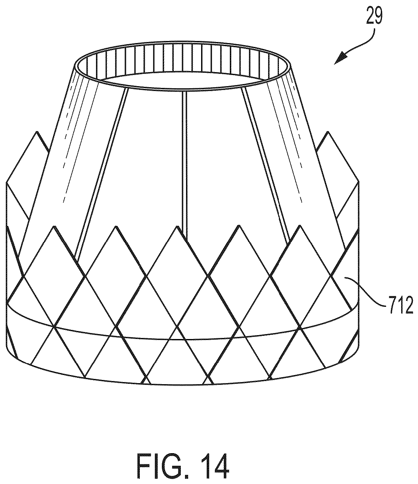

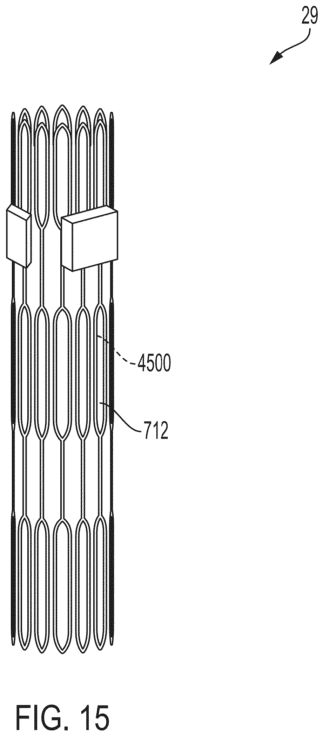

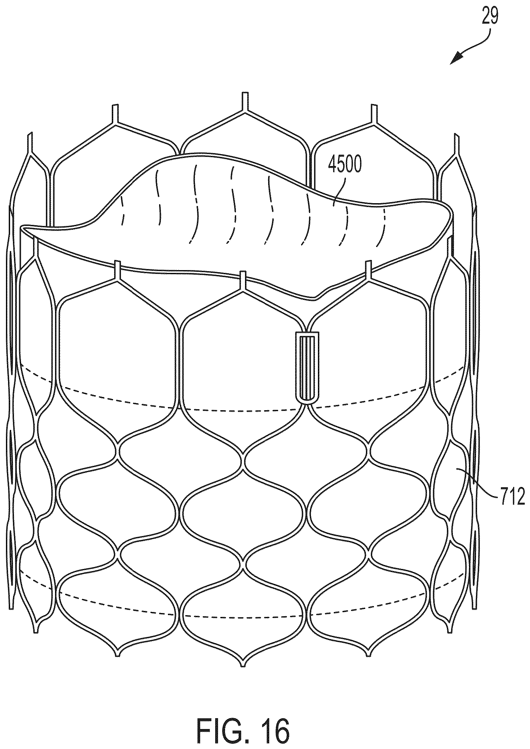

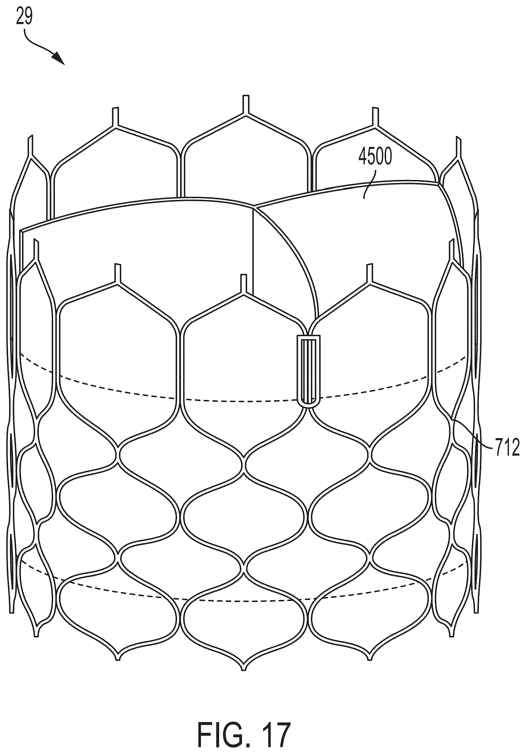

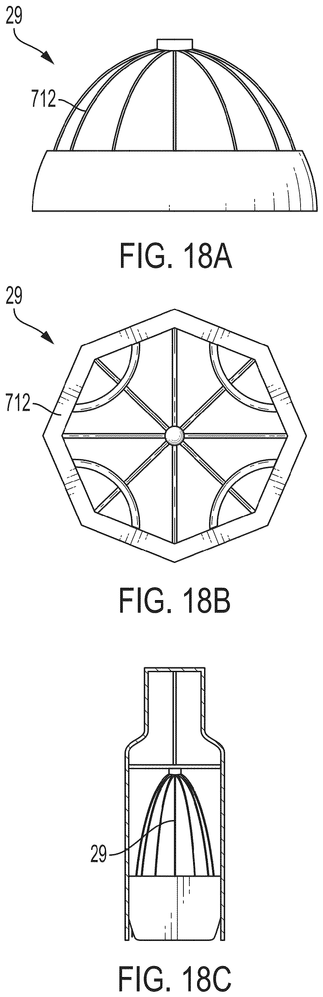

FIGS. 14 - 18C illustrate a few examples of the many valves or valve configurations that can be used. Any valve type can be used and some valves that are traditionally applied surgically can be modified for transcatheter implantation. FIG. 14 illustrates an expandable valve 29 for transcatheter implantation that is shown and described in US Patent No. 8,002,825, which is incorporated herein by reference in its entirety. An example of a tri-leaflet valve is shown and described in Published Patent Cooperation Treaty Application No. WO 2000/42950, which is incorporated herein by reference in its entirety. Another example of a tri-leaflet valve is shown and described in US Patent No. 5,928,281, which is incorporated herein by reference in its entirety. Another example of a tri-leaflet valve is shown and described in US Patent No. 6,558,418, which is incorporated herein by reference in its entirety. FIGS. 15 - 17 illustrate an exemplary embodiment of an expandable tri-leaflet valve 29, such as the Edwards SAPIEN Transcatheter Heart Valve. Referring to FIG. 15, in one exemplary embodiment the valve 29 comprises a frame 712 that contains a tri-leaflet valve 4500 (See FIG. 16) compressed inside the frame 712. FIG. 16 illustrates the frame 712 expanded and the valve 29 in an open condition. FIG. 17 illustrates the frame 712 expanded and the valve 29 in a closed condition. FIGS. 18A, 18B, and 18C illustrate an example of an expandable valve 29 that is shown and described in US Patent No. 6,540,782, which is incorporated herein by reference in its entirety. An example of a valve is shown and described in US Patent No. 3,365,728, which is incorporated herein by reference in its entirety. Another example of a valve is shown and described in US Patent No. 3,824,629, which is incorporated herein by reference in its entirety. Another example of a valve is shown and described in US Patent No. 5,814,099, which is incorporated herein by reference in its entirety. Any of these or other valves can be used as valve 29 in the various embodiments disclosed herein.

In still other embodiments (not shown), a valve may be integrally formed with the docking station, for example, by directly or integrally attaching valve leaflets (or any other suitable valving mechanism) to the valve seat

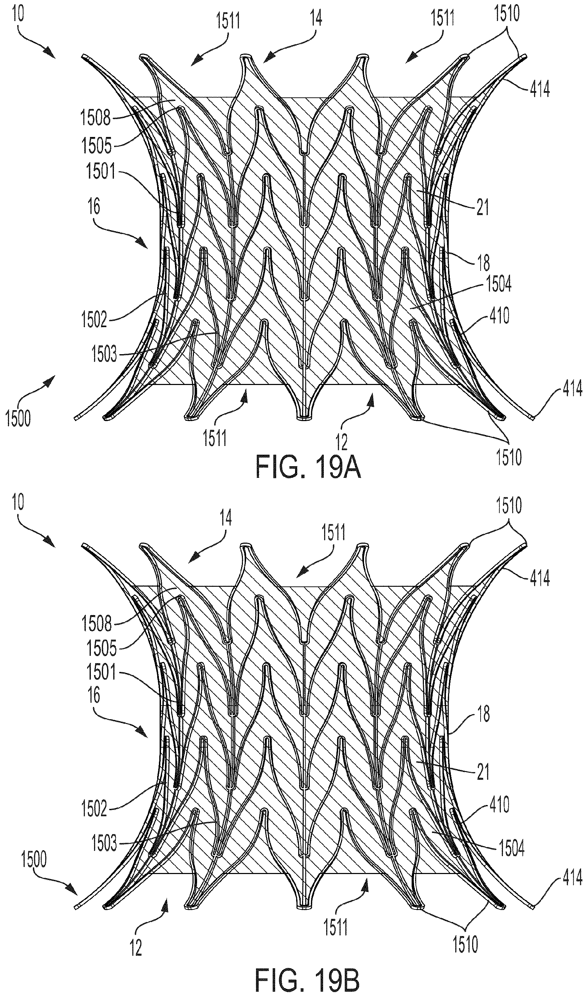

To provide for sealing engagement between the seal portions 410 and the inner surface 416 of the deployment site, and/or between the valve seat 18 and the valve 29, cloth, tissue, foam, or other such impermeable or substantially impermeable material 21 may be attached (e.g., sewed, stitched, sutured, or adhered) to the frame 1500. Referring now to FIGS. 19A through 19D, the cloth or impermeable material 21 can be cut, configured, or otherwise shaped such that the impermeable material 21 does not bunch and/or tear when the docking station 10 is compressed or deployed. In some such embodiments, the impermeable material 21 can be cut, configured, or otherwise shaped such that the impermeable material 21 does not cover at least a portion of the frame 1500 near the proximal end 12 and/or the distal end 14. The impermeable material 21 can be cut or shaped such that the impermeable material 21 does not cover at least a portion of the space not defined by one of the cells 1504 near the proximal and/or distal ends 12, 14. The impermeable material 21 can be configured or cut to the desired shape before the impermeable material 21 is attached to the frame 1500 or the impermeable material 21 can be attached to the frame 1500 and then cut to the desired shape.

At the proximal and distal ends 12, 14, the frame 1500 can include a plurality of openings 1511 between the strut portions 1502 and the apices 1510 in the portions of the frame 1500 which are not defined by the cells 1504. The openings 1511 are generally triangular in shape and are partially defined by two strut portions 1502, two apices 1510, and a junction 1503. The impermeable material 21 can be cut or shaped such that the impermeable material 21 does not cover at least a portion of the openings 1511 at the proximal and/or distal ends 12, 14.

The impermeable material 21 can be cut, configured, or otherwise shaped in a wide variety of ways such that the impermeable material 21 does not bunch or tear when the docking station 10 is compressed or deployed. The impermeable material 21 can be cut or shaped such that the impermeable material 21 can be attached to or disposed on the frame 1500 such that the impermeable material 21 can cover at least a portion of the cells 1504 but not cover at least a portion of the openings 1511 at the proximal and/or distal ends 12, 14.

As shown in FIG. 19A, the impermeable material 21 can be shaped or cut such that the impermeable material 21 substantially covers each cell 1504, substantially covers one-half of each opening 1511 at the proximal end 12, and substantially covers one-half of each opening 1511 at the distal end 14. However, the impermeable material 21 can be shaped or cut such that the impermeable material 21 substantially covers each cell 1504, substantially covers one-fourth, one-third, two-third, three-fourths, or any other suitable amount of each opening 1511 at the proximal end 12, and substantially covers one-fourth, one-third, two-third, three-fourths, or any other suitable amount of each opening 1511 at the distal end 14. Referring back to FIGS. 11A and 11B, the docking stations 10 can be used in differently sized circulatory system anatomies. By removing a portion of the material 21 in the opening 1511 at the proximal and/or distal end, the material 21 in the opening will not bunch up or the bunching up will be reduced when the docking station is used in a smaller circulatory system anatomy (e.g., FIG. 11B).

As shown in FIG. 19B, the impermeable material 21 substantially covers each cell 1504, substantially covers three-fourths of each opening 1511, at the proximal end 12 and generally does not cover the openings 1511 at the distal end 14. Additional material coverage at the proximal end may, for example, facilitate endothelialization at the inflow end of the docking station 10.

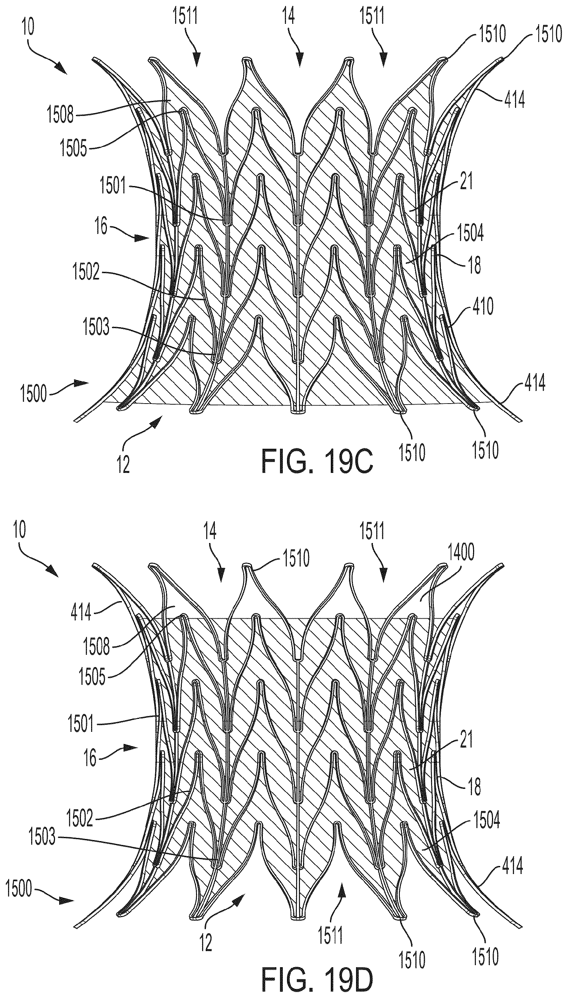

As shown in FIG. 19C, the impermeable material 21 substantially covers each cell 1504, substantially covers the openings 1511 at the proximal end 12, and generally does not cover the openings 1511 at the distal end 14.

In each of the illustrated embodiments, the impermeable material 21 is cut horizontally or straight across. However, the impermeable material 21 can be cut or shaped in any suitable direction or pattern. For example, the impermeable material 21 can be cut or shaped in a rounded or sinusoidal pattern. Additionally, the impermeable material 21 has been described as covering each of the openings 1511 at the proximal end 12 in a uniform manner and covering each of the openings 1511 at the distal end 14 in a uniform manner. However, the impermeable material 21 can be cut or shaped such that the openings 1511 at each end 12, 14 are not covered in a uniform manner. For example, each of the openings 1511 at either end 12, 14 can be covered in a different manner or amount than the other openings 1511. Further, the impermeable material 21 can be cut or shaped larger than desired such that the impermeable material 21 can be disposed on or affixed to the strut portions 1502, as detailed below.

The impermeable material 21 can also be cut or otherwise shaped such that the impermeable material 21 does not cover at least a portion of the distal most cells 1504 and/or the outflow cells 1508. In such an embodiment, a portion of the distal most cells 1504 or the outflow cells 1508 and the openings 1511 can form the permeable portion 1400. As shown in FIG. 19D, the impermeable material 21 can be cut or shaped such that the impermeable material 21 substantially covers the proximal most cells 1504, generally does not cover the openings 1511 at the proximal end 12, substantially covers one-half of each of the distal most cells 1504, and generally does not cover the openings 1511 at the distal end 14. The impermeable material 21 can be cut or otherwise shaped such that the impermeable material 21 extends horizontally across at a point substantially equivalent to the location of the distal most junctions 1503.

In the embodiment illustrated by FIG. 19D, the impermeable cover 21 substantially covers one-half of the distal most cells 1504, for example, to allow for perfusion. However, the impermeable cover 21 can cover any amount of the distal most cells 1504. For example, the impermeable cover 21 can be cut or shaped to cover one-fourth, one-third, two-third, three-fourths, or any other suitable amount of the distal most cells 1504. As another example, as shown in FIG. 10, the distal most cells may be entirely uncovered. In some such arrangements, the lack of cover material at the distal end may result in a docking station that provides a sealing portion 410 at only a proximal medial side of the docking station.

In the illustrated embodiment, the impermeable material 21 generally does not cover the openings 1511 at the proximal end 12. However, the impermeable material 21 can cover the openings 1511 at the proximal end 12 in any amount or manner, such as the ways depicted and described in FIGS. 19A, 19B, and 19C. Additionally, while the impermeable material 21 is depicted as extending horizontally across the distal most junctions, the impermeable material 21 can have any other suitable shape extending across the distal most cells 1504 and junctions 1503. For example, the impermeable material 21 can have a rounded, curved, sinusoidal, or any other cut or shape extending across the distal most cells 1504 and junctions 1503.

While the various configurations of the impermeable material 21 have been described and illustrated as being used with the frame 1500 of FIGS. 9A - 9D, the various configurations of the impermeable material 21 can be applied with any other docking station 10 described herein.

Still other features may be provided with the various docking stations and docking station frames of the present disclosure. For example, the docking station may be provided with radiopaque markers attached to the docking station (e.g., sewn into pouches in the impermeable material) for visualization/location of the docking station or a portion of the docking station (e.g., the waist portion) during or after deployment. Exemplary radiopaque markers and attachment arrangements are described in co-pending PCT application serial no. PCT/US2021/019770, the entire disclosure of which is incorporated herein by reference.

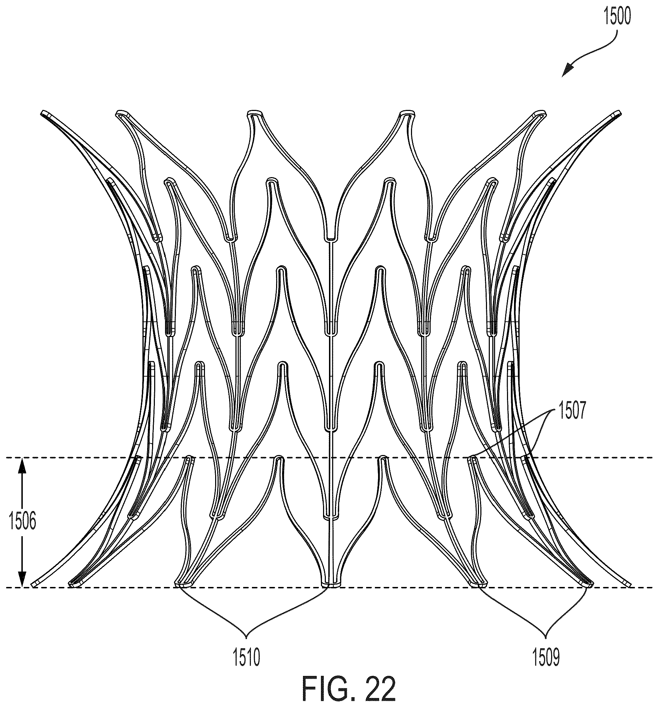

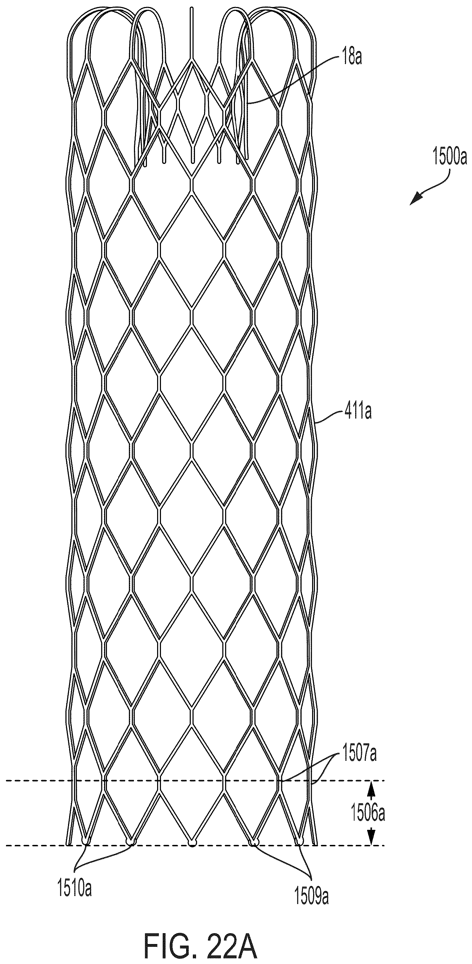

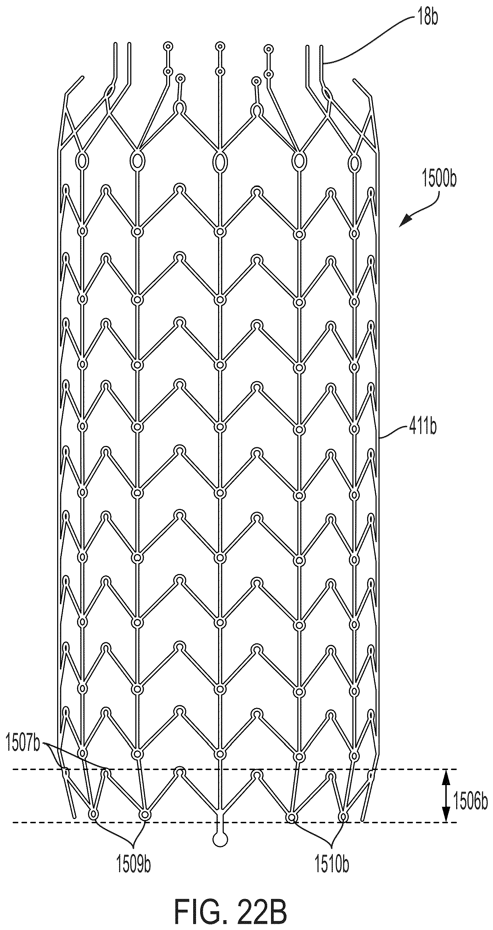

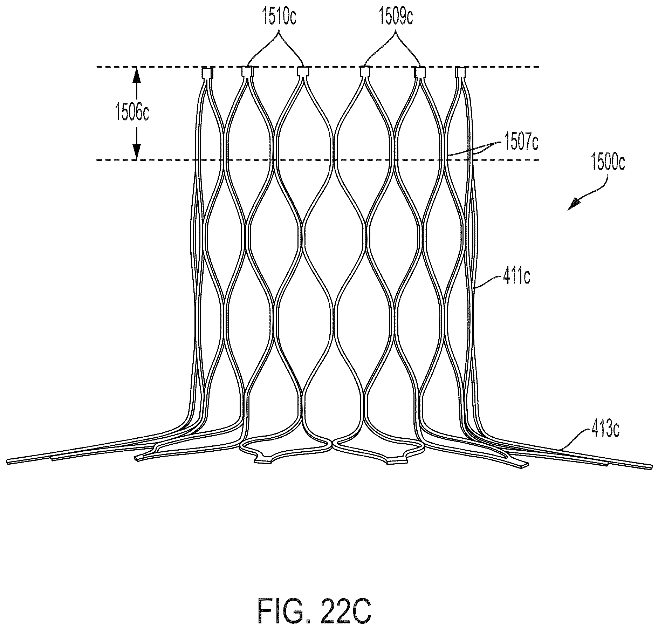

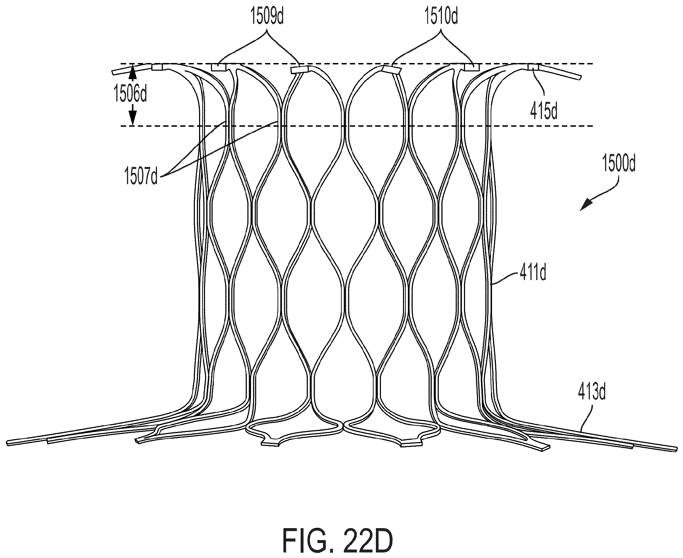

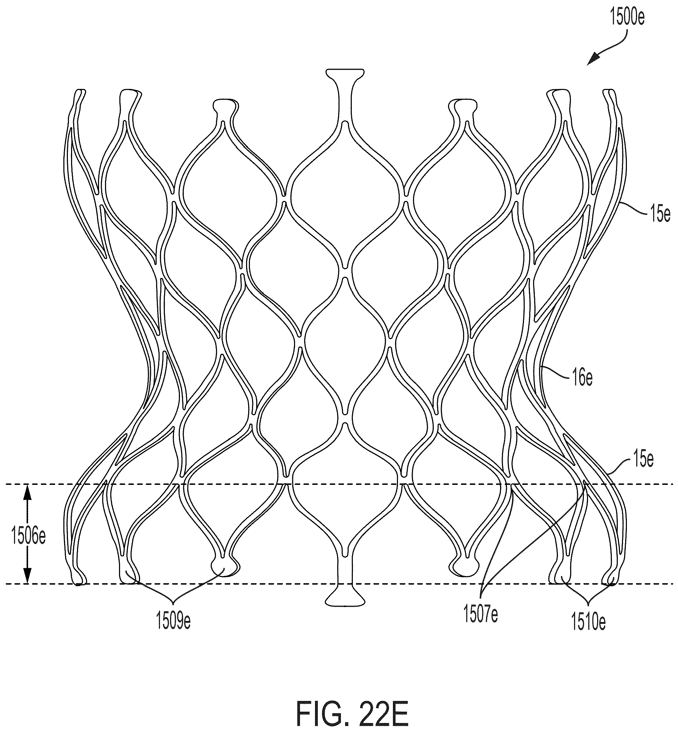

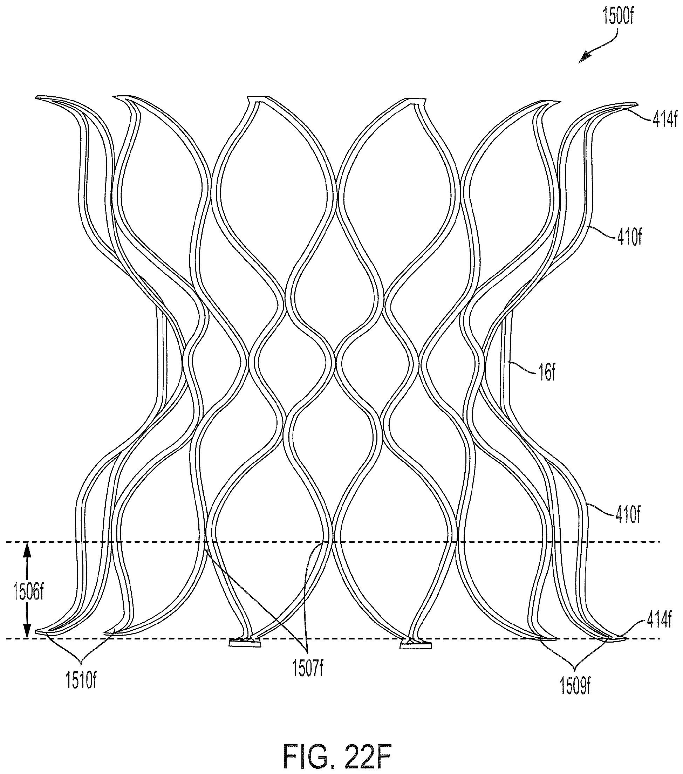

In various docking station frame embodiments and deployed arrangements, the outer profile contours of the docking station frame may vary, providing a variety of contours, lengths, and extents of retaining portion and sealing portion engagements between the frame and the vessel. FIGS. 22 and 22A-22F illustrate a variety of exemplary docking station frames 1500, 1500a-f.

In some exemplary arrangements, a docking station may include a docking station frame having a concave profile extending from flared retaining/sealing end portions to a narrower cylindrical or shallow concave waist portion defining a valve seat that may, but need not, be radially spaced from the vessel inner surface. FIG. 22 illustrates one such exemplary docking station frame 1500 including a concave profile extending from flared retaining/sealing end portions 414 to a narrower cylindrical or shallow concave waist portion 16 defining a valve seat 18, as also illustrated in FIGS. 9A-9D, 10, and 19A-19D and described above.

In other exemplary arrangements, a docking station may include an elongated cylindrical shaped docking station frame providing a substantially uniform retaining and sealing profile in the expanded, unconstrained state, with radially inward extending legs supporting a valve seat radially inward of the cylindrical retaining/sealing profile portions. FIGS. 22A and 22B illustrate exemplary docking station frames 1500a, 1500b including a cylindrical shaped outer frame portion 411a, 411b with radially inward offset valve seat portion 18a, 18b disposed at a distal or second end of the frame 1500a, 1500b. Similar docking station implementations are shown and described in greater detail in co-owned PCT Application Pub. No. WO 2022/040120, the entire disclosure of which is incorporated herein by reference.

In other exemplary arrangements, a docking station may include an elongated cylindrical shaped docking station frame providing a substantially uniform valve seat defining portion in the expanded, unconstrained state, with radially outward extending flexible flanged portions defining retaining and sealing portions at one or both ends of the frame. FIGS. 22C and 22D illustrate exemplary docking station frames 1500c, 1500d having an elongated cylindrical portion 411c, 411d and radially outward extending flexible flanged portions 413c, 413d, 415d at one (FIG. 22C) or both (FIG. 22D) ends of the frame. Similar docking station implementations are shown and described in greater detail in co-owned PCT Application Pub. No. WO 2022/103734, the entire disclosure of which is incorporated herein by reference.

In other exemplary arrangements, a docking station may include an hourglass shaped docking station frame, with convex profiled end portions providing tangential retaining and sealing engagement with the vessel inner surface, and a concave profiled central or waist portion defining a valve seat radially spaced from the vessel inner surface. FIG. 22E illustrates an exemplary hourglass shaped docking station frame 1500e with convex end portions 15e and a concave waist portion 16e. Similar docking station implementations are shown and described in greater detail in co-owned PCT Application Pub. No. WO 2022/103734, the entire disclosure of which is incorporated herein by reference.

In other exemplary arrangements, a docking station may include an hourglass shaped docking station frame, with flared profiled end portions providing flared retaining engagement with the vessel inner surface, a concave profiled central or waist portion defining a valve seat radially spaced from the vessel inner surface, and convex profiled sealing portions between the waist portion and the flared end portions providing tangential sealing engagement with the vessel inner surface. FIG. 22F illustrates an exemplary hourglass shaped docking station frame 1500f with flared end (retaining) portions 414f, convex medial (sealing) portions 410f, and a concave central waist portion 16f. Similar docking station implementations are shown and described in greater detail in co-owned U.S. Patent No. 10,363,130, the entire disclosure of which is incorporated herein by reference.

In some embodiments and applications, engagement profiles between the docking station frame 1500, 1500a-f and the inner surface 416 may vary, depending, for example, on docking station frame size, shape, and flexibility and inner vessel size, shape, and pliability.

In embodiments utilizing a docking station frame having a substantially continuous curved longitudinal profile, such as the docking station frame 1500 of FIG. 22, deployment of the docking station 10 at an implantation site in the circulatory system (e.g., the pulmonary artery) causes the radially flared retaining portions 414 to engage the inner surface of the vessel. In some applications, an interference fit between the expanded docking station frame 1500 and the inner surface 416 causes the ends of the docking station frame to flex radially inward, exerting a radially outward force at the retaining portions 414 and potentially creating radial gaps between the docking station frame 1500 and the inner surface 416 at locations between the retaining portions 414 and the sealing portions 410. Additionally, the substantially continuous curved longitudinal profile of the docking station frame 1500 may result in a radial gap between the waist portion 16 and the inner surface 416.

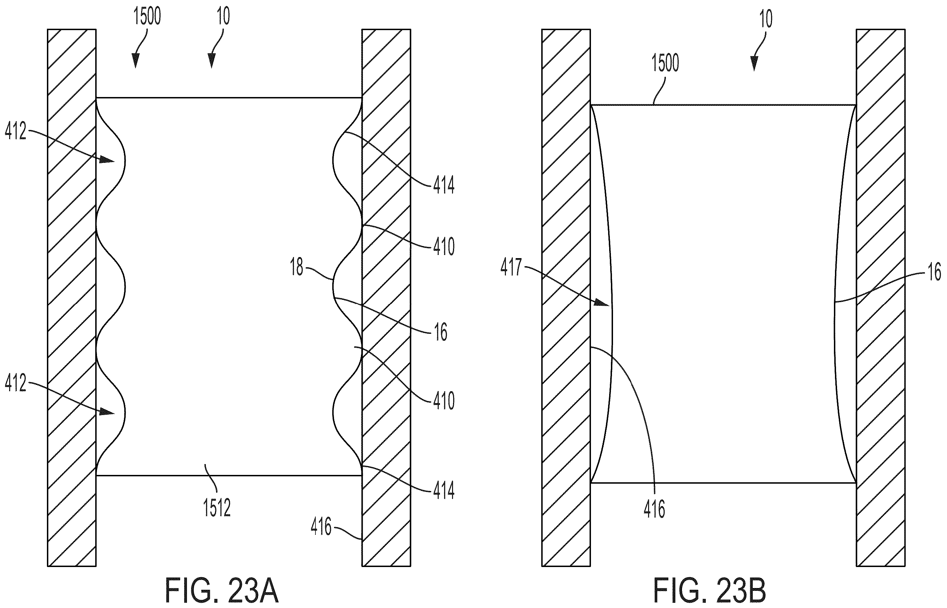

In some such implementations, as schematically illustrated in FIG. 23A, radially outward flared retaining end portions 414 engage the inner surface 416 of the vessel deployment site, and are separated from rounded or convex sealing medial portions 410 by radial gaps 412 between the docking station frame 1500 and the inner surface 416, with a radial gap between the central waist portion 16 and the inner surface 416. This engagement profile may also be provided by other docking station frame configurations, including, for example, flared ended hourglass shaped frames, such as, for example, the docking station frame 1500f of FIG. 22F, and exemplary embodiments of the above incorporated U.S. Patent No. 10,363,130.

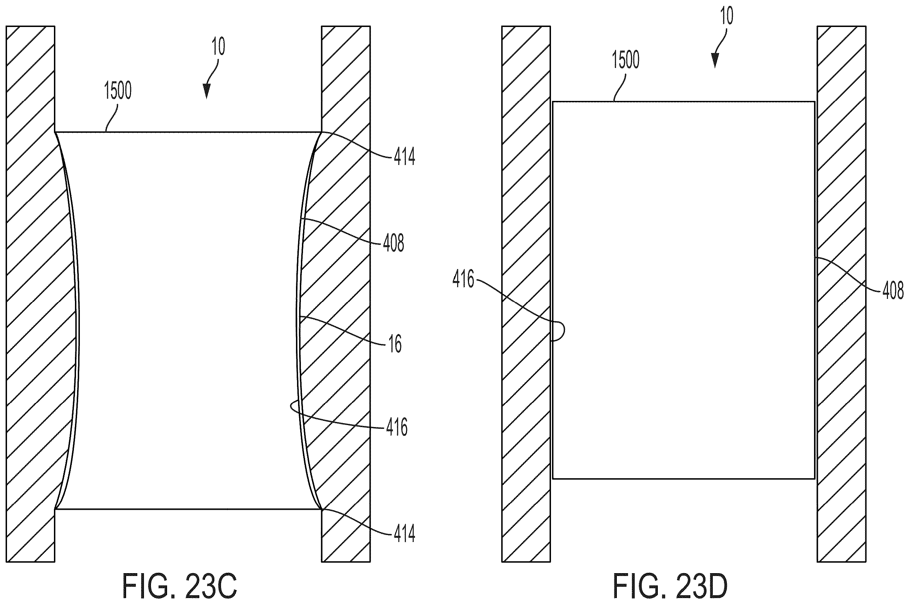

In other applications a docking station frame having a substantially continuous curved longitudinal profile, such as the docking station frame 1500 of FIG. 22, the deployed, inner vessel constricted frame may maintain a substantially concave profile along its length, resulting in flared retaining engagement at the end portions or apices, an extended concave sealing profile axially inward from the end portions, and a central shallow concave or cylindrical waist portion defining the valve seat. In some applications, as shown in FIG. 23B, the concave profile 408 of the frame 1500 may provide a radial gap 417 between the vessel inner surface 416 and the waist portion 16. In other applications, as shown in FIG. 23C, the waist portion 16 of the frame 1500 may contact the vessel inner surface.

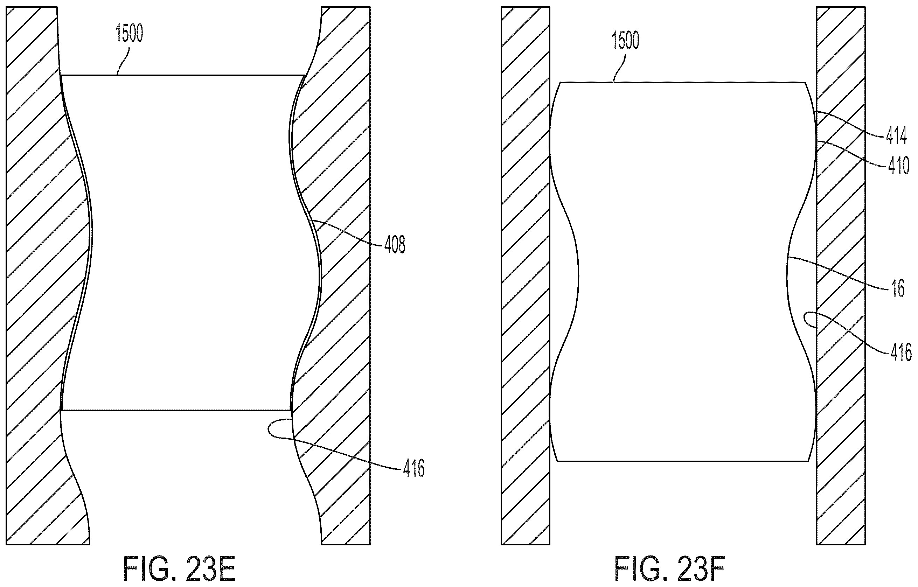

In other exemplary implementations, substantially continuous and/or uniform engagement may be provided along the length of the docking station frame 1500, for example, with the vessel and docking station frame having corresponding straight/cylindrical profiles 408, as schematically shown in FIG. 23D, or with the docking station frame 1500 variably expanding to closely conform with a non-cylindrical vessel, as schematically shown in FIG. 23E. Such an engagement profile may be provided, for example, by docking station frames having cylindrical shaped outer portions with radially inward offset valve seat portions, such as, for example, the docking station frames 1500a, 1500b of FIGS. 22A and 22B, and exemplary embodiments of the above incorporated PCT Application Pub. No. WO 2022/040120.

In other exemplary implementations, as schematically shown in FIG. 23F, a docking station frame 1500 is expanded into unflared engagement at the retaining end portions 414 which gradually transition to unflared tangential engagement (e.g., cylindrical, convex, concave) at the sealing medial portions 410, with a radial gap between the concave or cylindrical central waist portion 16 and the inner surface 416. Such an engagement profile may be provided, for example, by hourglass shaped docking station frames having convex end portions, such as, for example, the docking station frame 1500e of FIG. 22E, and exemplary embodiments of the above incorporated PCT Application Pub. No. WO 2022/103734.

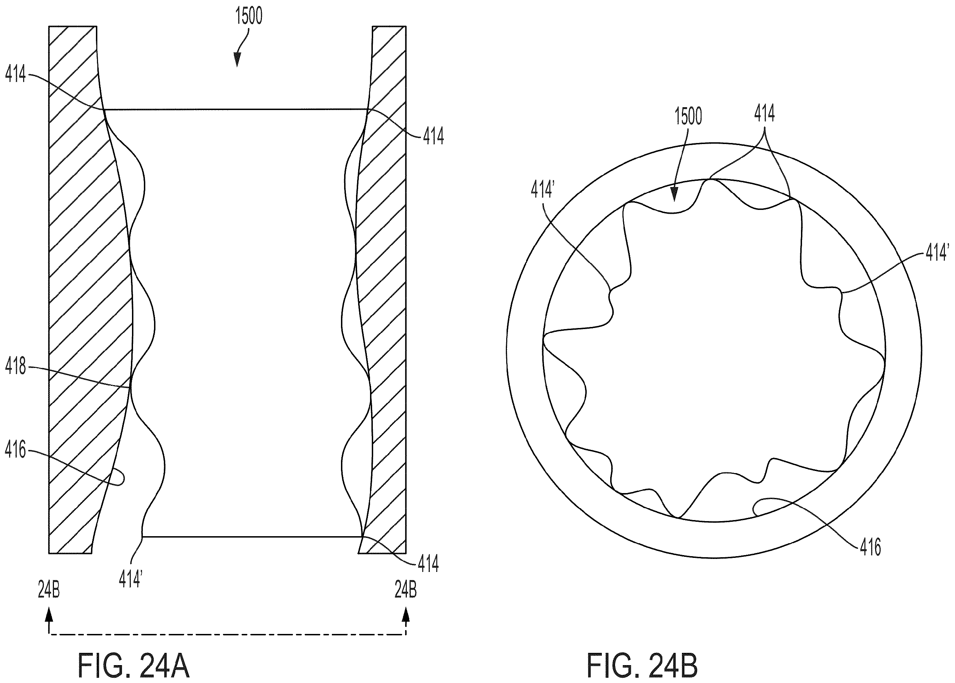

In some applications, for example, due to the non-cylindrical shape of the pulmonary artery or other deployment site, one or more of the retaining portion apices may not contact the vessel inner surface, for example, during the systolic phase when lower blood pressure provides less pressure against the retaining portions. In some such applications, as schematically shown in FIGS. 24A and 24B, one or more of the frame end retaining portions 414' may extend radially inward to varying degrees, and out of engagement with the vessel inner surface 416, for example, due to constriction of portions 418 of the frame 1500 axially inward of frame end 1512. In such an arrangement, radially inward extending apices may obstruct an end portion of the central opening of the docking station frame, and may interfere with a valve installation tube or catheter inserted into the docking station frame to install a prosthetic valve at the docking station valve seat.

According to an exemplary aspect of the present disclosure, a docking station for a prosthetic valve may be provided with a plurality of radiopaque markers around a first, insertion or catheter receiving end of the docking station frame, to provide visual identification of the locations of the docking station frame first end, around a first end opening of the frame, to facilitate insertion of a catheter or other such device into the first end opening without contacting the docking station frame first end. The frame can include a plurality of radiopaque markers spaced circumferentially around the first end of the docking station frame to establish an annular demarcation at the first end opening of the docking station.

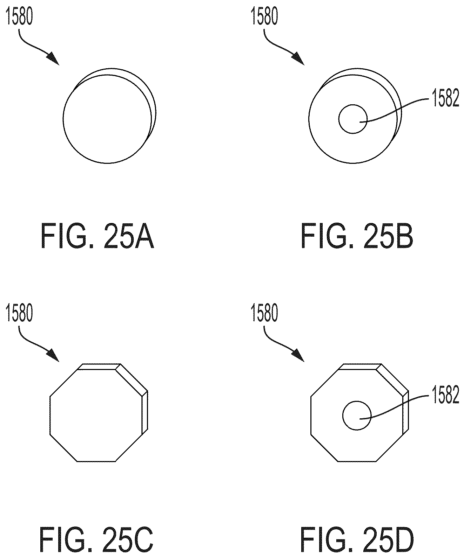

The plurality of radiopaque markers can be radiopaque or have a higher radiopacity such that the one or more radiopaque markers can be identified under fluoroscopy or a similar imaging process. The plurality of radiopaque markers can be disposed on, attached to, integrated with, or otherwise affixed to the docking station in a wide variety of arrangements or configurations, such as the arrangements or configurations detailed below. The plurality of radiopaque markers can comprise any material or combination of materials that are radiopaque or increase the radiopacity of at least a portion of the catheter receiving end of the docking station. For example, the plurality of radiopaque markers can comprise barium sulfate, bismuth, tungsten, tantalum, platinum-iridium, gold, or any other material which is opaque to fluoroscopy, X-rays, or similar radiation or any combination thereof. As illustrated in FIGS. 25A-25D, the radiopaque markers 1580 may be thin and flat, or disc-shaped, and may be provided in a variety of shapes, including, for example circular or octagonal, as shown. The one or more radiopaque markers 1580 can be configured to facilitate secure attachment against axial movement on the frame, and can be any suitable shape. For example, the one or more radiopaque markers 1580 can be hexagonal, triangular, rectangular, elliptical, semicircular, three-dimensionally contoured (i.e., non-flat), or any other shape or configuration. The radiopaque markers 1580 can also include an aperture 1582 extending through a central portion of the marker 1580. The aperture 1582 can be sized such that a suture can pass therethrough. Exemplary radiopaque markers for use with docking station frames are described in co-owned PCT Application Pub. No. WO 2021/188278, the entire disclosure of which is incorporated herein by reference.

As used herein, for docking station frames having an endmost zigzag shaped portion (e.g., the endmost zigzag shaped portions 1506, 1506a-f of the docking station frames 1500, 1500a-f of FIGS. 22 and 22A-F), an "end" of a docking station frame (e.g., first end, second end, catheter receiving end, insertion end) may include the portion of the frame 1500 1500a-f extending axially outward from the inner axial junctions 1507, 1507a-f of the zigzag shaped portion. In some implementations, this endmost portion of the frame may be most susceptible to variability in radial placement during implantation (and resulting variations in the size and shape of the frame end opening), for example, due to varying constriction, bending, or flexing by the vessel inner surface.

The docking station frame may be provided with radiopaque markers at a variety of locations at the first end of the docking station frame, including, for example, at the inner axial junctions of the endmost zigzag shaped portion of the frame, at the outer axial junctions of the endmost zigzag shaped portion of the frame, or at the endmost apices of the first end of the frame (which may, but need not, coincide with the outer axial junctions of the endmost zigzag shaped portion of the frame), or at any location between the inner axial junctions, the outer axial junctions, and the endmost apices. In some implementations, the radiopaque markers are disposed axially outward of an axial midpoint between the inner axial junctions and the outer axial junctions.

In some exemplary arrangements, radiopaque markers are mechanically affixed to the catheter receiving end of the frame. In certain implementations, the radiopaque markers 1580 can be attached or affixed to the struts or junctions at the catheter receiving first end of the frame, with the radiopaque markers 1580 affixed to the frame 1500 in any suitable manner. For example, the radiopaque markers 1580 can be affixed to the frame 1500 by an adhesive, a suture, press fit, snap fit, or any other suitable arrangement. In some implementations, a docking station frame may be provided with a plurality of marker seating portions (e.g., apertures, recesses) circumferentially spaced around the first end of the docking station frame. The marker seating portions are sized to receive and retain the corresponding plurality of radiopaque markers (e.g., by adhesive, press fit, snap fit, etc.). The marker seating portions may be provided at a variety of locations on the struts at the first end of the frame, including, for example, the inner axial junctions 1507, 1507a-f of the endmost zigzag portion 1506, 1506a-f, the outer axial junctions 1509, 1509a-f of the endmost zigzag shaped portion, and the endmost apices 1510, 1510a-f, any of which may be provided with a thicker portion of frame material to accommodate a recessed or apertured marker seating portion. Exemplary radiopaque marker seating configurations are described in the above incorporated PCT Application Pub. No. WO 2021/188278.

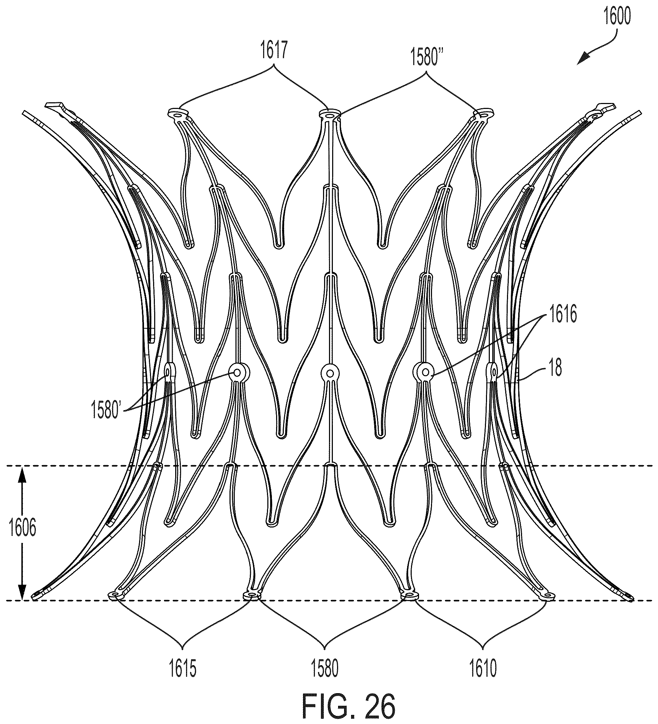

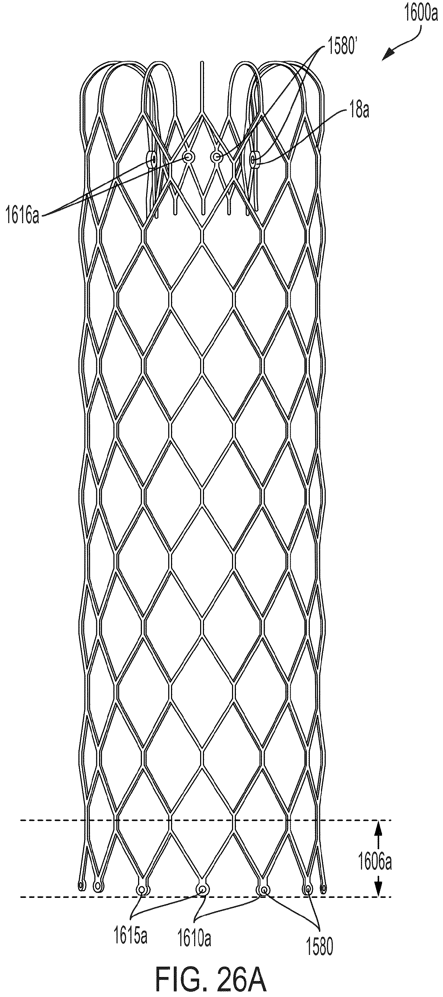

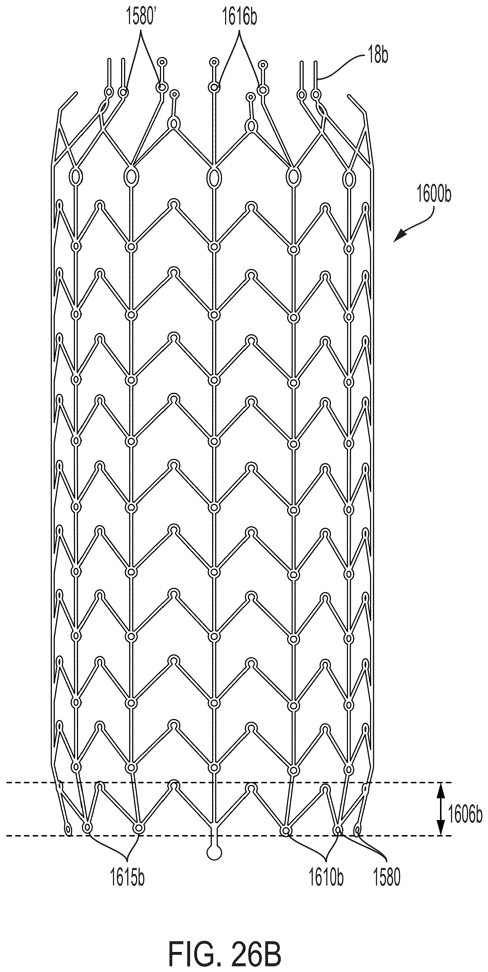

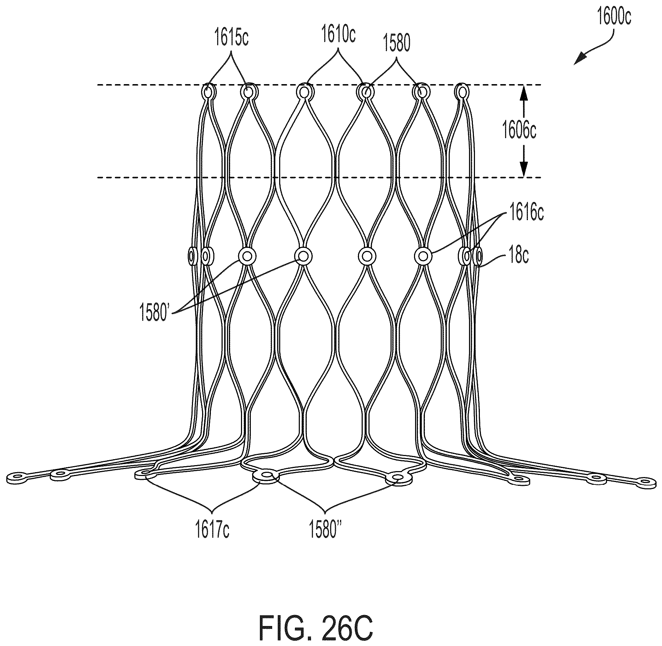

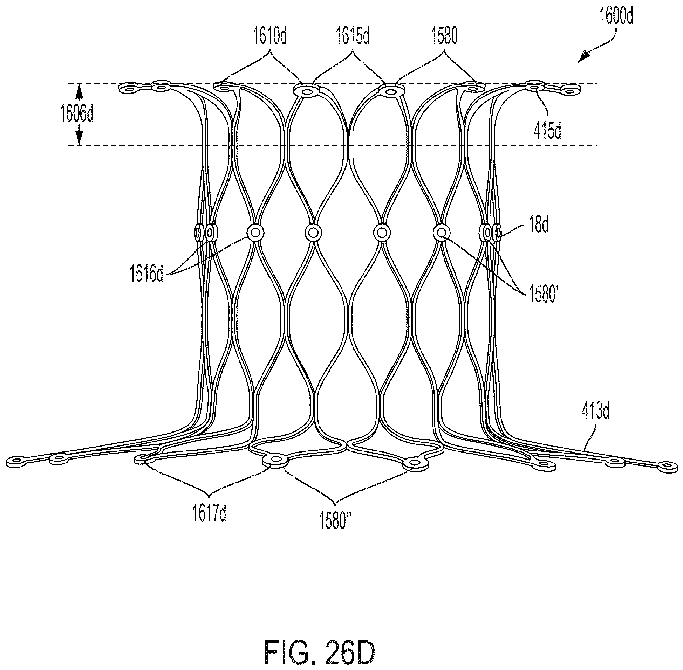

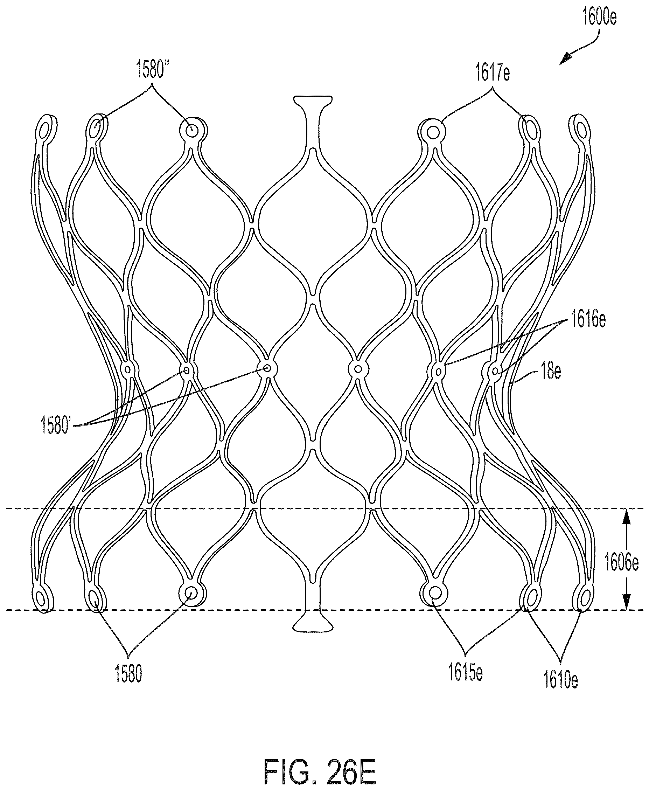

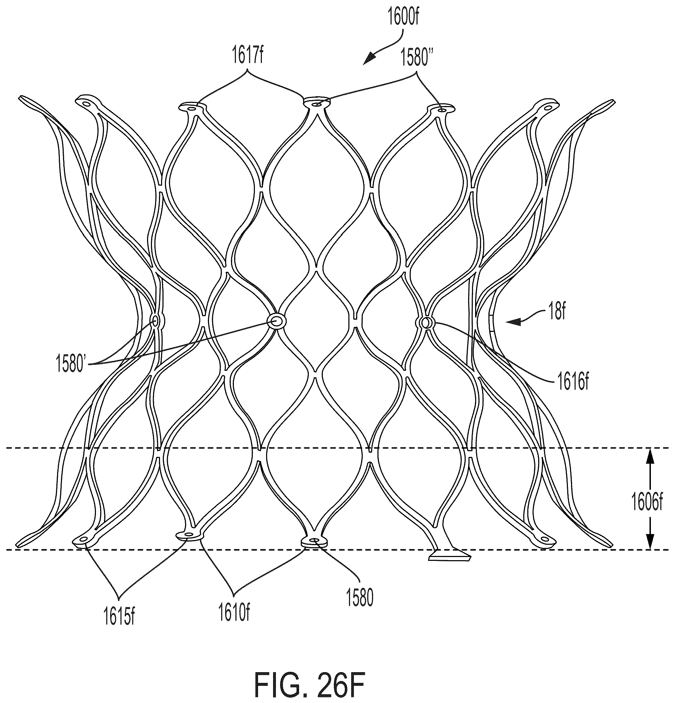

FIGS. 26 and 26A-F illustrate exemplary docking station frames 1600, 1600a-f, similar to the docking station frames 1500, 1500a-f of FIGS. 22 and 22A-22F, with a first end 1606, 1606a-f (e.g., catheter receiving end) of the docking station frame provided with endmost apices 1610, 1610a-fhaving apertured marker seating portions 1615, 1615a-f retaining radiopaque markers 1580 in seating engagement (e.g., by adhesive, press fit, snap fit, etc.).

In other exemplary implementations, instead of being separate pieces which are affixed to the frame, the radiopaque markers 1580 are included in or integrally formed with the frame (e.g., at the first end apices 1610, 1610a-f of the frames 1600, 1600a-f of FIGS. 26 and 26A-F). The radiopaque markers can be built into the frame, or the radiopaque markers can be formed as thicker frame junctions in the first or catheter receiving end of the frame which increases the radiopacity or radiodensity first end of the frame. For example, in embodiments where the frame comprises nitinol, additional nitinol can be deposited at the first end frame junctions or apices in to increase the radiopacity or radiodensity of the frame end. However, the radiopacity or radiodensity of the frame end can be increased in a variety of other ways, such as by depositing additional and/or different radiopaque materials at one or more frame junctions or apices of the frame end.

In other exemplary arrangements, radiopaque markers are attached or affixed to a material secured to the docking station frame, such as, for example, a seal-forming impermeable material secured (e.g., sewed, sutured, adhered) to the frame (as further described elsewhere in the disclosure), with the radiopaque markers attached or affixed to a portion of the material disposed on the first end of the frame. For example, the radiopaque markers can be affixed to first end locations of the impermeable material or other frame secured material by an adhesive, a suture, a pocket, or any other suitable arrangement. In some implementations, a docking station frame may be provided with a material at least partially disposed at the first end of the frame, with marker attachment locations circumferentially spaced on the secured material around the first end of the docking station frame. The marker attachment locations may be provided at a variety of locations at the first end of the frame, including, for example, anywhere between the inner axial junctions of the endmost zigzag portion, the outer axial junctions of the endmost zigzag shaped portion, and the endmost apices. Exemplary radiopaque marker attachment arrangements for frame secured material are described in the above incorporated PCT Application Pub. No. WO 2021/188278.

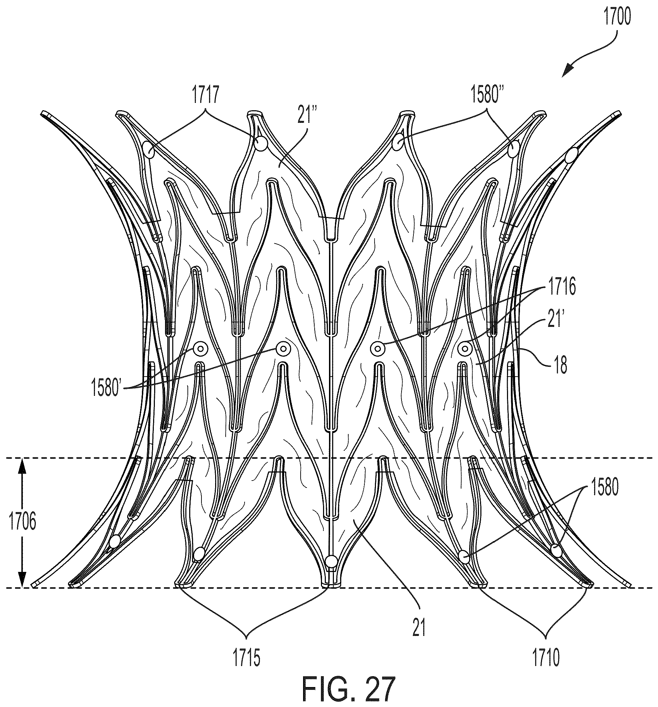

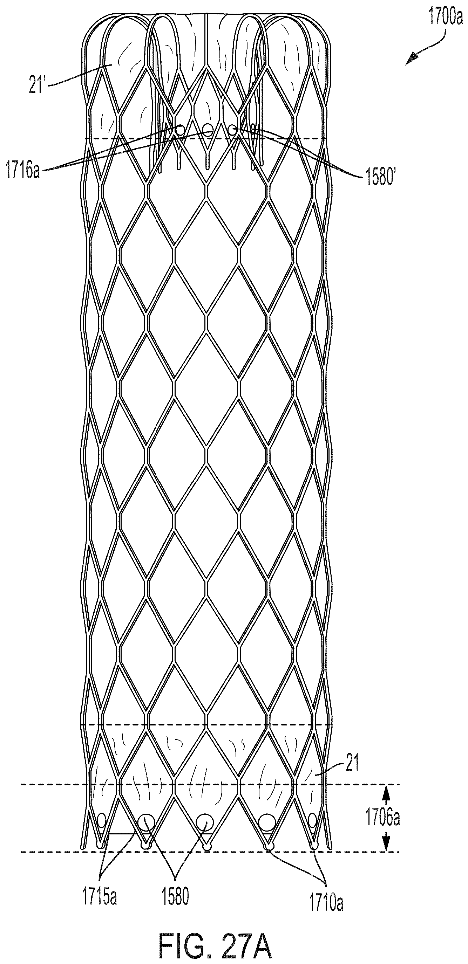

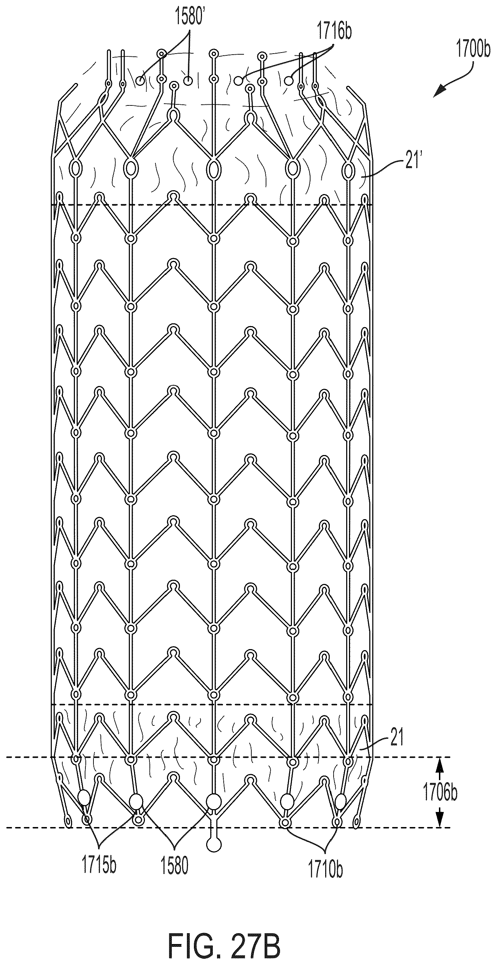

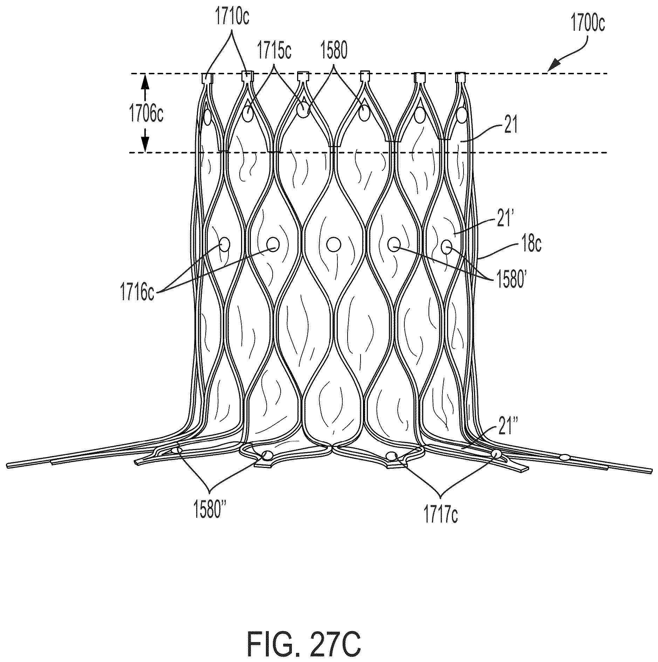

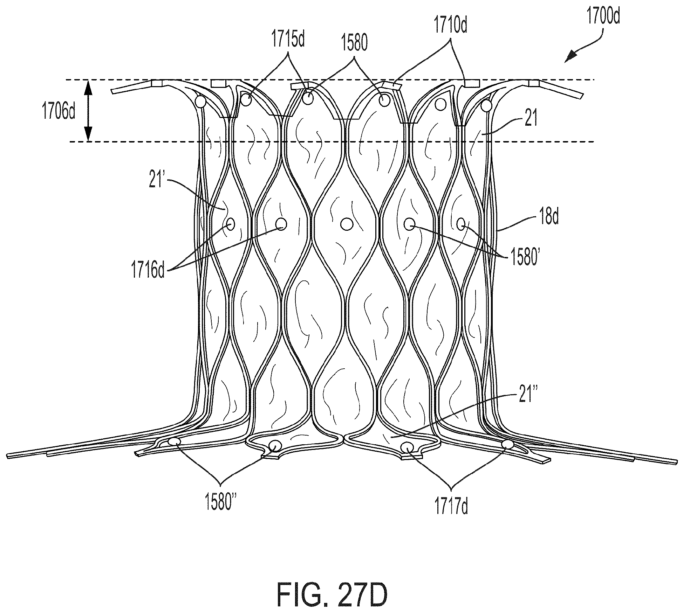

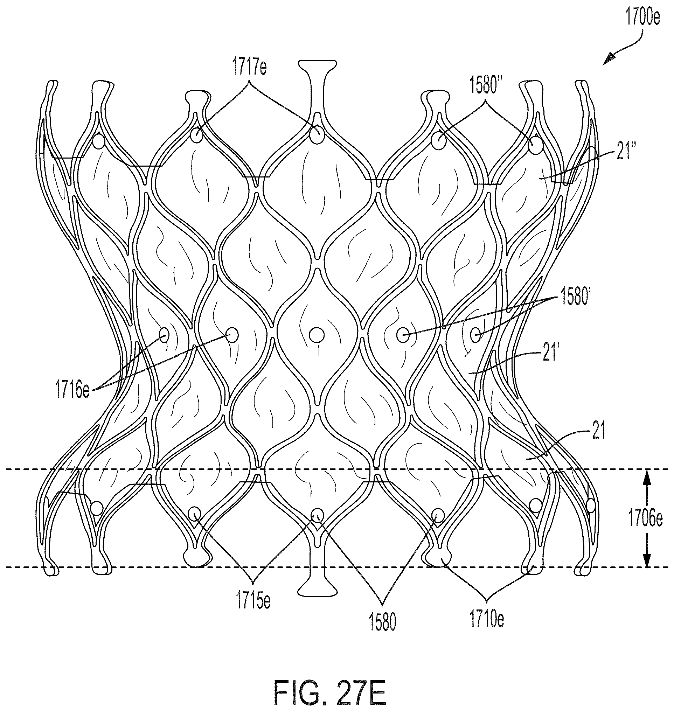

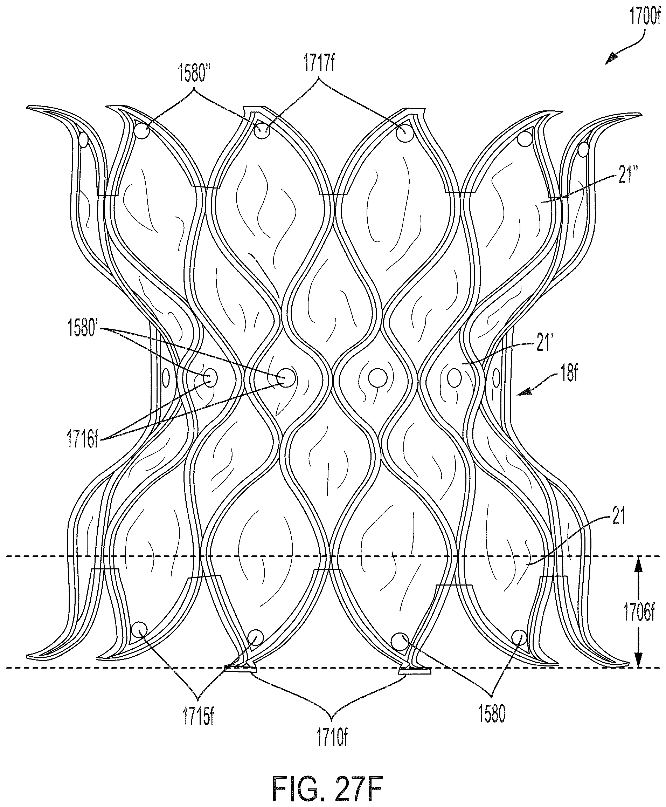

FIGS. 27 and 27A-F illustrate exemplary docking station frames 1700, 1700a-f, similar to the docking station frames 1500, 1500a-f of FIGS. 22 and 22A-F, with a first end 1706, 1706a-f (e.g., catheter receiving end) of the docking station frame provided with material 21 (e.g., impermeable fabric or other frame cover material, such as, for example, the impermeable cover material 21 of FIGS. 19A-19D) secured (e.g., sewed, sutured, adhered) to the frame, and radiopaque markers 1580 attached to the material 21 at circumferentially spaced marker attachment locations 1715, 1715a-f (e.g., proximate the apices 1710, 1710a-f). Various arrangements and methods for securing an impermeable cover material to a docking station frame are described in the above incorporated PCT Application Pub. No. WO 2021/188278.

In some implementations, an additional set of one or more radiopaque markers may be provided on the docking station frame at or near the valve seat, for example, to assist with deployment of the docking station as well as placement of the valve into the valve seat. The additional set of radiopaque markers may, but need not, be of similar construction to the first set of radiopaque markers provided at the first end of the docking station frame, as described above. In certain implementations, the radiopaque markers can be attached or affixed to the struts or junctions in the valve seat of the frame, with the radiopaque markers affixed to the frame in any suitable manner. For example, the radiopaque markers can be affixed directly to the frame, for example, by an adhesive, a suture, press fit, snap fit, integral formation with the frame struts, or any other suitable means. Alternatively, the radiopaque markers can be affixed to material secured to the second end of the frame. The frame can include any number of radiopaque markers spaced circumferentially around the valve seat to establish an annular plane through the valve seat of the docking station.

In the exemplary embodiments of FIGS. 26 and 26A-F, the docking station frames 1600, 1600a-f include marker seating portions 1616, 1616a-f retaining radiopaque markers 1580' (e.g., by adhesive, press fit, snap fit, etc.) at strut junctions aligned with or proximate to the valve seat 18, 18a-f. Alternatively, the radiopaque markers 1580' at the valve seat 18, 18a-f may be included in or integrally formed with the frame 1600, 1600a-f.

In the exemplary embodiments of FIGS. 27 and 27A-F, the docking station frames 1700, 1700a-f include material 21' (e.g., impermeable fabric or other frame cover material, such as, for example, the impermeable cover material 21 of FIGS. 19A-19D) secured (e.g., sewed, sutured, adhered) to the frame at the valve seat 18, 18a-f, and radiopaque markers 1580' attached to the material 21' at circumferentially spaced marker attachment locations 1716, 1716a-f at or proximate to the valve seat. The material 21' may be separate from material 21 at the first end 1706, 1706a-f of the frame 1700, 1700a-f (to which first end markers 1580 are attached), or part of the same material extending from the frame first end to at least the valve seat.

In some implementations, an additional set of one or more radiopaque markers may be provided on the second end of docking station frame (e.g., between the inner axial junctions and the end apices of the endmost zigzag portion of the frame second end, for example, to assist with deployment or orientation of the docking station, or to facilitate installation of a valve or other tool insertion into the second end of the frame. The additional set of radiopaque markers may, but need not, be of similar construction to the first set of radiopaque markers provided at the first end of the docking station frame, as described above. In certain implementations, the radiopaque markers can be attached or affixed to the struts or junctions in the valve seat of the frame, with the radiopaque markers affixed to the frame in any suitable manner. For example, the radiopaque markers can be affixed directly to the frame, for example, by an adhesive, a suture, press fit, snap fit, integral formation with the frame struts, or any other suitable means. Alternatively, the radiopaque markers can be affixed to material secured to the second end of the frame. The frame can include any number of radiopaque markers spaced circumferentially around the frame second end to identify the perimeter of the frame second end opening.

In the exemplary embodiments of FIGS. 26 and 26C-F, the docking station frames 1600, 1600a-finclude second end apices with marker seating portions 1617, 1617c-f retaining radiopaque markers 1580'' (e.g., by adhesive, press fit, snap fit, etc.). Alternatively, the radiopaque markers 1580'' at the frame second end may be included in or integrally formed with the frame 1600, 1600c-f.

In the exemplary embodiments of FIGS. 27 and 27C-F, the docking station frames 1700, 1700c-f include material 21" (e.g., impermeable fabric or other frame cover material, such as, for example, the impermeable cover material 21 of FIGS. 19A-19D) secured (e.g., sewed, sutured, adhered) to the frame at the second end, and radiopaque markers 1580'' attached to the material 21" at circumferentially spaced marker attachment locations 1717, 1717c-f at the frame second end. The material 21" may be separate from material 21 at the first end 1706, 1706c-f of the frame 1700, 1700c-f (to which first end markers 1580 are attached), or part of the same material extending from the frame first end to at least the valve seat.

In some implementations, the docking station frame may be provided with a first set of radiopaque markers at the frame first end and a second set of radiopaque markers at or near the valve seat. In some implementations, the docking station frame may be provided with a first set of radiopaque markers at the frame first end and a second set of radiopaque markers at the frame second end. In some implementations, the docking station frame may be provided with a first set of radiopaque markers on the frame first end, a second set of radiopaque markers at or near the valve seat, and a third set of radiopaque markers at the frame second end.

In some implementations, the sets of radiopaque markers may be arranged or configured to visually distinguish between first, second, and (when in use) third sets of radiopaque markers. For example, the different sets of radiopaque markers may be selected, configured, or arranged to have distinguishable different marker size, marker shape, marker orientation, and/or distance between adjacent markers around the circumference of the frame.

Methods of treating a patient (e.g., methods of treating heart valve dysfunction/regurgitation, etc.), or carrying out a simulation of treating a patient, can include a variety of steps, including steps associated with introducing and deploying a docking station in a desired location/treatment area and introducing and deploying a valve in the docking station.

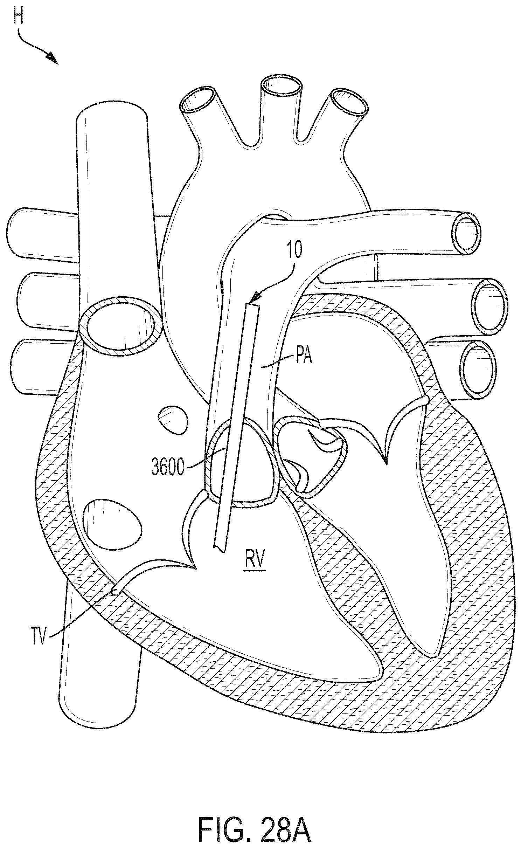

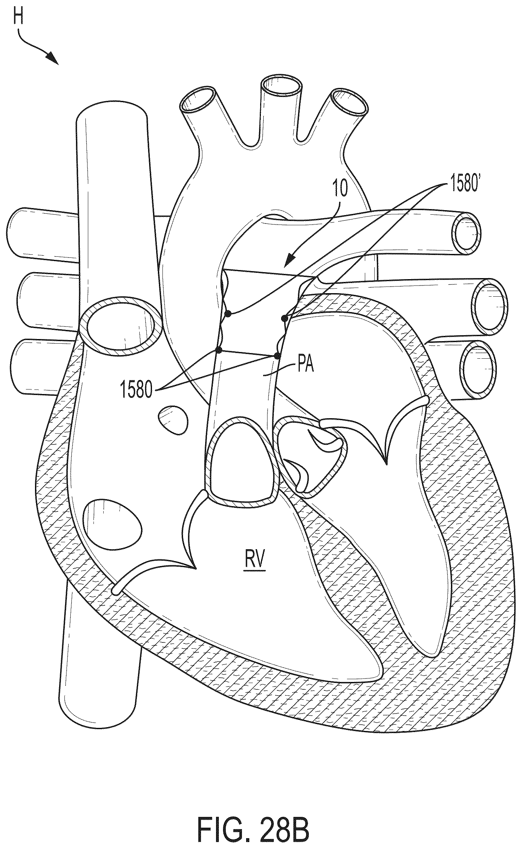

For example, FIG. 28A illustrates a docking station 10 (e.g., any of the docking stations utilizing any of the docking station frames 1500 described herein) being deployed by a first catheter 3600 including a first tube retaining the docking station prior to deployment. In one exemplary embodiment, a guidewire followed by a catheter 3600 is advanced to the pulmonary artery PA by way of the femoral vein, inferior vena cava, tricuspid valve, and right ventricle RV. When deployed, as shown in FIG. 28B, the docking station frame 1500 expands at the target location, with the proximal first end of the docking station frame expanding radially outward of the valve seat to engage the inner surface of the pulmonary artery PA, to retain the docking station frame at the target location. The first catheter is then withdrawn from the pulmonary artery. Such withdrawal may be facilitated by visually confirming a positional arrangement of the first end of the expanded docking station frame by visually identifying locations of the plurality of radiopaque markers 1580.

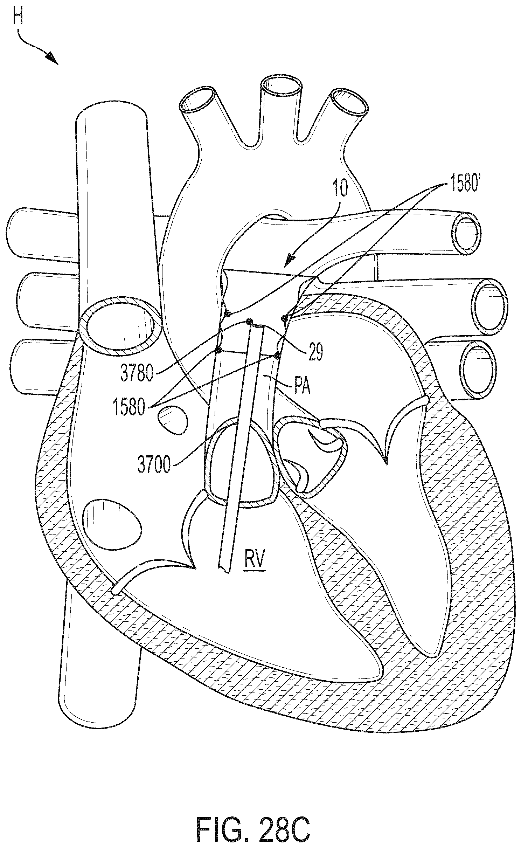

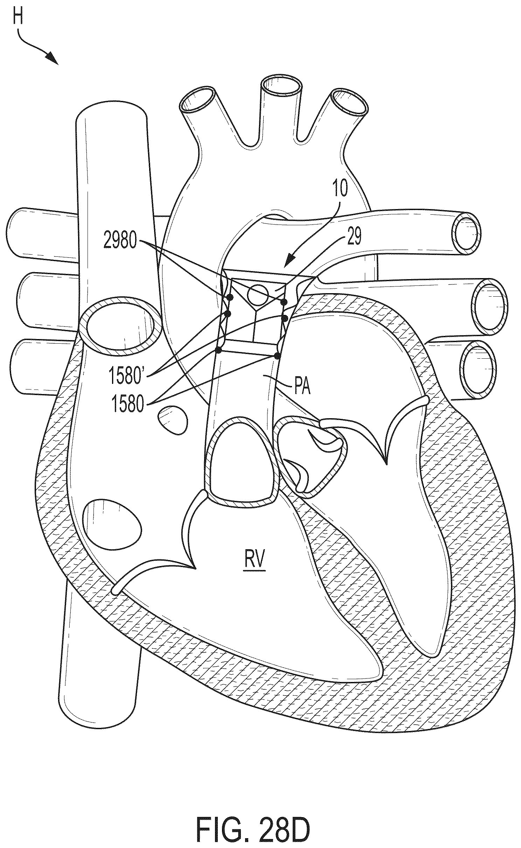

A second catheter 3700 with a second tube retaining an expandable prosthetic valve in a compressed condition is then inserted into the pulmonary artery PA. A terminal end of the second tube is extended between the visually identified locations of the plurality of radiopaque markers and into the first end of the expanded docking station frame (FIG. 28C). The expandable prosthetic valve 29 is deployed from the terminal end of the second tube and expanded into seating engagement with the valve seat 18 of the expanded docking station frame (FIG. 28D). The alignment of the deployed valve 29 with the valve seat 18 may be facilitated by visually confirming a location of the valve seat by visually identifying locations of a second plurality of radiopaque markers 1580' disposed on the docking station frame at or near the valve seat. Additionally, one or more radiopaque markers may be provided on at least one of the prosthetic valve 29 (e.g., at 2980 in FIG. 28D) and the second tube terminal end (e.g., at 3780 in FIG. 28C) to further facilitate alignment of the deployed valve with the valve seat by visual confirmation of the position of the second tube terminal end or prosthetic valve with respect to the valve seat. Exemplary arrangement of radiopaque markers on catheter tubes are described in the above incorporated PCT Application Pub. No. WO 2021/188278.

The foregoing primarily describes embodiments of docking stations that are self-expanding. But the docking stations and/or delivery devices shown and described herein can be modified for delivery of balloon-expandable and/or mechanically expandable docking devices, within the scope of the present disclosure. That is to say, delivering balloon-expandable and/or mechanically expandable docking stations to an implantation location can be performed percutaneously using modified versions of the delivery devices of the present disclosure. In general terms, this includes providing a transcatheter assembly that can include a delivery sheath and/or additional sheaths as described above. In the case of balloon-expandable docking stations, the devices generally further include a delivery catheter, a balloon catheter, and/or a guide wire. A delivery catheter used in a balloon-expandable type of delivery device can define a lumen within which the balloon catheter is received. The balloon catheter, in turn, defines a lumen within which the guide wire is slidably disposed. Further, the balloon catheter includes a balloon that is fluidly connected to an inflation source. With the docking station mounted on the balloon, the transcatheter assembly is delivered through a percutaneous opening in the patient via the delivery device. Once the docking station is properly positioned, the balloon catheter is operated to inflate the balloon, thus transitioning the docking station to an expanded arrangement.

### Examples

Example 1. A docking station frame for a medical device, the docking station frame comprising a plurality of strut portions extending from a proximal end to a distal end and forming a plurality of cells, wherein when the docking station frame is in an expanded, unconstrained condition, the plurality of strut portions form a substantially continuous curved longitudinal profile having outer radial retaining portions at the proximal and distal ends and an inner radial central portion defining a valve seat.

Example 2. The docking station frame of Example 1, wherein the plurality of strut portions comprise a plurality of circumferential strut portions axially spaced to define a plurality of rows of the plurality of cells, and a plurality of longitudinal strut portions circumferentially spaced and joined with the plurality of circumferential strut to define the cells of the plurality of cells in each of the plurality of rows.

Example 3. The docking station frame of Example 2, wherein the plurality of circumferential strut portions comprises at least five circumferential strut portions defining at least four rows of the plurality of cells.

Example 4. The docking station frame of any of Examples 2 and 3, wherein the plurality of longitudinal strut portions comprises at least twelve longitudinal strut portions defining at least twelve cells in each of the plurality of rows.

Example 5. The docking station frame of any of Examples 2 - 4, wherein the plurality of circumferential strut portions comprises undulating strut portions.

Example 6. The docking station frame of Example 5, wherein the parallel undulating strut portions are substantially parallel and shaped such that each of the plurality of cells is substantially herringbone shaped.

Example 7. The docking station frame of any of Examples 5 and 6, wherein a distal one of the parallel undulating strut portions defines the outer radial retaining portion at the distal end, and a proximal one of the parallel undulating strut portions defines the outer radial retaining portion at the proximal end.

Example 8. The docking station frame of any of Examples 5 - 7, wherein each of the plurality of undulating strut portions includes proximal portions joined with a corresponding one of the plurality of longitudinal strut portions, and distal portions unattached from the plurality of longitudinal strut portions.

Example 9. The docking station frame of any of Examples 1 - 8, wherein when the docking station frame is in the expanded, unconstrained condition, the waist has a diameter of about 27 mm and is expandable to accommodate a valve having a diameter of about 29 mm.

Example 10. The docking station frame of any of Examples 1 - 9, wherein when the docking station frame is in the expanded, unconstrained condition, the docking station frame has a length between about 35 mm and about 40 mm.

Example 11. The docking station frame of any of Examples 1 - 10, wherein when the docking station frame is in the expanded, unconstrained condition, the retaining portions extend at an angle between about 30° and about 45° with respect to a central longitudinal axis of the docking station frame.

Example 12. The docking station frame of any of Examples 1 - 11, wherein when the docking station frame is in the expanded, unconstrained condition, the waist portion is substantially cylindrical.

Example 13. The docking station frame of any of Examples 1 - 12, wherein when the docking station frame is in the expanded, unconstrained condition, the retaining portions have a radius of curvature of between about 14 mm and about 20 mm.

Example 14. The docking station frame of any of Examples 1-13, wherein when the docking station frame is in the expanded, unconstrained condition, the inner radial central portion is contoured to provide a valve seat having a length of at least about 8 mm for a valve having a deployed outer diameter of 29 mm.

Example 15. The docking station frame of any of Examples 1-14, wherein the docking station frame is configured to be crimped to a diameter of no greater than about 5.4 mm.

Example 16. A docking station frame for a medical device, the docking station frame comprising a plurality of strut portions extending from a proximal end to a distal end and forming a plurality of cells, wherein when the docking station frame is in an expanded, unconstrained condition, the plurality of strut portions form outer radial retaining portions at the proximal and distal ends extending at an angle between about 30° and about 45° with respect to a central longitudinal axis of the docking station frame.

Example 17. The docking station frame of Example 16, wherein the plurality of strut portions comprise a plurality of circumferential strut portions axially spaced to define a plurality of rows of the plurality of cells, and a plurality of longitudinal strut portions circumferentially spaced and joined with the plurality of circumferential strut to define the cells of the plurality of cells in each of the plurality of rows.

Example 18. The docking station frame of Example 17, wherein the plurality of circumferential strut portions comprises at least five circumferential strut portions defining at least four rows of the plurality of cells.

Example 19. The docking station frame of any of Examples 17 and 18, wherein the plurality of longitudinal strut portions comprises at least twelve longitudinal strut portions defining at least twelve cells in each of the plurality of rows.

Example 20. The docking station frame of any of Examples 17 - 19, wherein the plurality of circumferential strut portions comprises undulating strut portions.

Example 21. The docking station frame of Example 20, wherein the parallel undulating strut portions are substantially parallel and shaped such that each of the plurality of cells is substantially herringbone shaped.

Example 22. The docking station frame of any of Examples 20 and 21, wherein a distal one of the parallel undulating strut portions defines the outer radial retaining portion at the distal end, and a proximal one of the parallel undulating strut portions defines the outer radial retaining portion at the proximal end.

Example 23. The docking station frame of any of Examples 20 - 22, wherein each of the plurality of undulating strut portions includes proximal portions joined with a corresponding one of the plurality of longitudinal strut portions, and distal portions unattached from the plurality of longitudinal strut portions.

Example 24. The docking station frame of any of Examples 16 - 23, wherein when the docking station frame is in the expanded, unconstrained condition, the waist has a diameter of about 27 mm and is expandable to accommodate a valve having a diameter of about 29 mm.

Example 25. The docking station frame of any of Examples 16 - 24, wherein when the docking station frame is in the expanded, unconstrained condition, the docking station frame has a length between about 35 mm and about 40 mm.

Example 26. The docking station frame of any of Examples 16 - 25, wherein when the docking station frame is in the expanded, unconstrained condition, the inner radial central portion is substantially cylindrical.

Example 27. The docking station frame of any of Examples 16 - 26, wherein when the docking station frame is in the expanded, unconstrained condition, the retaining portions have a radius of curvature of between about 14 mm and about 20 mm.

Example 28. The docking station frame of any of Examples 16 - 27, wherein when the docking station frame is in the expanded, unconstrained condition, the inner radial central portion is contoured to provide a valve seat having a length of at least about 8 mm for a valve having a deployed outer diameter of 29 mm.

Example 29. The docking station frame of any of Examples 16 - 28, wherein the docking station frame is configured to be crimped to a diameter of no greater than about 5.4 mm.

Example 30. A docking station frame for a medical device, the docking station frame comprising a plurality of strut portions extending from a proximal end to a distal end and forming a plurality of cells, wherein when the docking station frame is in an expanded, unconstrained condition, the plurality of strut portions form a curved longitudinal profile having outer radial retaining portions at the proximal and distal ends and an inner radial central portion defining a valve seat, the inner radial central portion being substantially cylindrical and the outer radial retaining portions each having a radius of curvature between about 14 mm and about 20 mm.

Example 31. The docking station frame of Example 30, wherein the plurality of strut portions comprise a plurality of circumferential strut portions axially spaced to define a plurality of rows of the plurality of cells, and a plurality of longitudinal strut portions circumferentially spaced and joined with the plurality of circumferential strut to define the cells of the plurality of cells in each of the plurality of rows.

Example 32. The docking station frame of Example 31, wherein the plurality of circumferential strut portions comprises at least five circumferential strut portions defining at least four rows of the plurality of cells.

Example 33. The docking station frame of any of Examples 31 and 32, wherein the plurality of longitudinal strut portions comprises at least twelve longitudinal strut portions defining at least twelve cells in each of the plurality of rows.

Example 34. The docking station frame of any of Examples 31 - 33, wherein the plurality of circumferential strut portions comprises undulating strut portions.

Example 35. The docking station frame of Example 34, wherein the parallel undulating strut portions are substantially parallel and shaped such that each of the plurality of cells is substantially herringbone shaped.

Example 36. The docking station frame of any of Examples 34 and 35, wherein a distal one of the parallel undulating strut portions defines the outer radial retaining portion at the distal end, and a proximal one of the parallel undulating strut portions defines the outer radial retaining portion at the proximal end.

Example 37. The docking station frame of any of Examples 34 - 36, wherein each of the plurality of undulating strut portions includes proximal portions joined with a corresponding one of the plurality of longitudinal strut portions, and distal portions unattached from the plurality of longitudinal strut portions.

Example 38. The docking station frame of any of Examples 30 - 37, wherein when the docking station frame is in the expanded, unconstrained condition, the waist has a diameter of about 27 mm and is expandable to accommodate a valve having a diameter of about 29 mm.

Example 39. The docking station frame of any of Examples 30 - 38, wherein when the docking station frame is in the expanded, unconstrained condition, the docking station frame has a length between about 35 mm and about 40 mm.

Example 40. The docking station frame of any of Examples 30 - 39, wherein when the docking station frame is in the expanded, unconstrained condition, the retaining portions extend at an angle between about 30° and about 45° with respect to a central longitudinal axis of the docking station frame.

Example 41. The docking station frame of any of Examples 30 - 40, wherein when the docking station frame is in the expanded, unconstrained condition, the retaining portions have a maximum diameter of about 44 mm.

Example 42. The docking station frame of any of Examples 30 - 41, wherein when the docking station frame is in the expanded, unconstrained condition, the inner radial central portion is contoured to provide a valve seat having a length of at least about 8 mm for a valve having a deployed outer diameter of 29 mm.

Example 43. The docking station frame of any of Examples 30 - 42, wherein the docking station frame is configured to be crimped to a diameter of no greater than about 5.4 mm.

Example 44. A docking station for a medical device, the docking station comprising:
the docking station frame of any of Examples 1-43; and
an impermeable material attached to the docking station frame.

Example 45. The docking station of Example 44, wherein the impermeable material is attached to the valve seat to provide a seal between the valve seat and a valve installed in the valve seat.

Example 46. The docking station of any of Examples 44 and 45, wherein the impermeable material is attached to a medial portion of the docking station frame between the inner radial central portion and at least one of the outer radial retaining portions to provide a medial sealing portion for sealing with a deployment site in a circulatory system when the docking station is deployed at the deployment site.

Example 47. A system comprising:
a delivery catheter including a tube;
a docking station disposed in the tube, wherein the docking station comprises the docking station of any of Examples 44 - 46.

Example 48. A docking station for a prosthetic valve, the docking station comprising:
an expandable docking station frame including a plurality of struts extending from a first end to a second end of the docking station frame, the expandable frame further defining a valve seat for securing a prosthetic valve to the expandable frame;
wherein the first end of the docking station frame defines a retaining portion expandable radially outward of the valve seat to engage an inner surface of a circulatory system at a deployed position over a range of sizes of expansion; and
a plurality of radiopaque markers disposed around the first end of the docking station frame.

Example 49. The docking station of Example 48, wherein the first end of the docking station frame includes an endmost zigzag shaped portion of the docking station frame, alternating between outer axial apices and inner axial junctions.

Example 50. The docking station of Example 49, wherein each of the plurality of radiopaque markers is located between the inner axial junctions and the outer axial apices.

Example 51. The docking station of Example 50, wherein each of the plurality of radiopaque markers is disposed on a corresponding one of the plurality of the outer axial apices.

Example 52. The docking station of Example 51, wherein each of the plurality of radiopaque markers is attached to the corresponding one of the plurality of the outer axial apices.

Example 53. The docking station of any of Examples 48-52, wherein the docking station frame includes a plurality of integrally formed marker seating portions each retaining one of the plurality of radiopaque markers therein.

Example 54. The docking station of any of Examples 48-52, further comprising an impermeable material attached to the docking station frame and including a first end portion disposed at a first end of the docking station frame, wherein each of the plurality of radiopaque markers is affixed to the first end portion of the impermeable material.

Example 55. The docking station of any of Examples 48-51, wherein each of the plurality of radiopaque markers is integrally formed with the first end of the docking station frame.

Example 56. The docking station of Example 55, wherein the plurality of radiopaque markers comprises enlarged junctions between the plurality of struts.

Example 57. The docking station of any of Examples 48-56, further comprising a second plurality of radiopaque markers disposed around a second axial location of the docking station frame, axially spaced from the first end of the docking station frame.

Example 58. The docking station of Example 57, wherein the second plurality of radiopaque markers is disposed around the valve seat.

Example 59. The docking station of Example 57, wherein the second plurality of radiopaque markers is disposed around the second end of the docking station frame.

Example 60. The docking station of Example 59, wherein the second plurality of radiopaque markers is disposed between outer axial apices and inner axial junctions of the second end of the docking station frame.

Example 61. The docking station of Example 60, wherein the second plurality of radiopaque markers is disposed on a plurality of the outer axial apices of the second end of the docking station frame.

Example 62. The docking station of Example 61, wherein the second plurality of radiopaque markers is attached to the plurality of the outer axial apices of the second end of the docking station frame.

Example 63. The docking station of any of Examples 57-62, wherein the docking station frame includes a second plurality of integrally formed marker seating portions each retaining one of the second plurality of radiopaque markers therein.

Example 64. The docking station of any of Examples 57-62, further comprising an impermeable material attached to the docking station frame, wherein each of the second plurality of radiopaque markers is affixed to the impermeable material.

Example 65. The docking station of any of Examples 57-61, wherein each of the second plurality of radiopaque markers is integrally formed with the docking station frame.

Example 66. The docking station of Example 65, wherein the second plurality of radiopaque markers comprises enlarged junctions between the plurality of struts.

Example 67. The docking station of any of Examples 57-66, wherein each of the first plurality of radiopaque markers has a first configuration, and each of the second plurality of radiopaque markers has a second configuration different from the first configuration.

Example 68. The docking station of Example 67, wherein the second configuration differs from the first configuration in at least one of: marker size, marker shape, marker orientation, and distance between adjacent markers.

Example 69. The docking station of any of Examples 48-68, wherein the valve seat is disposed on a medial portion of the docking station frame between the first and second ends.

Example 70. The docking station of any of Examples 48-69, wherein the docking station frame defines a concave profile extending from the first and second ends to a narrower cylindrical or shallow concave waist portion.

Example 71. The docking station of any of Examples 48-69, wherein the docking station frame defines a cylindrical outer portion, with the valve seat being offset radially inward from the outer portion.

Example 72. The docking station of any of Examples 48-69, wherein the docking station frame includes at least one radially outward extending flanged end portion.

Example 73. The docking station of any of Examples 48-69, wherein the docking station frame includes convex profiled first and second end portions and a concave waist portion defining the valve seat.

Example 74. The docking station of any of Examples 48-69, wherein the docking station frame includes flared first and second end portions and a concave waist portion defining the valve seat.

Example 75. A method of installing a prosthetic valve in a vessel, the method comprising:
providing an expandable docking station frame in a compressed condition within a first tube;
inserting the first tube into the vessel;
deploying the docking station from the first tube and expanding the docking station frame at a target location within the vessel, such that a first end of the docking station frame expands radially outward of the valve seat to engage an inner surface of the vessel to retain the docking station frame at the target location;
withdrawing the first tube from the vessel;
providing an expandable prosthetic valve in a compressed condition within a second tube;
inserting the second tube into the vessel;
visually confirming a positional arrangement of the first end of the expanded docking station frame by visually identifying locations of a plurality of radiopaque markers disposed around the first end of the docking station frame;
extending a terminal end of the second tube between the visually identified locations of the plurality of radiopaque markers and into the first end of the expanded docking station frame; and
deploying the expandable prosthetic valve from the terminal end of the second tube and expanding the prosthetic valve into seating engagement with a valve seat of the expanded docking station frame.

Example 76. The method of Example 75, wherein the docking station further comprises a second plurality of radiopaque markers disposed around a second axial location of the docking station frame, axially spaced from the first end of the docking station frame.

Example 77. The method of Example 75, wherein deploying the docking station frame from the first tube and expanding the docking station frame at the target location within the vessel comprises partially deploying the docking station frame from the first tube and visually confirming locations of the second plurality of radiopaque markers at the target location before fully deploying the docking station frame the docking station frame from the first tube.

Example 78. The method of any of Examples 76-77, wherein the second plurality of radiopaque markers are disposed around the valve seat, and wherein deploying the expandable prosthetic valve from the terminal end of the second tube comprises visually confirming locations of the second plurality of radiopaque markers in alignment with the expandable prosthetic valve before fully deploying the expandable prosthetic valve.

Example 79. The method of Example 78, wherein at least one of the prosthetic valve and the second tube terminal end includes at least one radiopaque marker, wherein visually confirming the locations of the second plurality of radiopaque markers in alignment with the expandable prosthetic valve comprises visually confirming the locations of the second plurality of radiopaque markers in alignment with the at least one radiopaque marker.

Example 80. The method of any of Examples 75-79, wherein the docking station comprises the docking station of any of Examples 48-74.

In view of the many possible embodiments to which the principles of the disclosed invention may be applied, it should be recognized that the illustrated embodiments are only preferred examples of the invention and should not be taken as limiting the scope of the invention. All combinations or sub-combinations of features of the foregoing exemplary embodiments are contemplated by this application. The scope of the invention is defined by the following claims. We therefore claim as our invention all that comes within the scope and spirit of these claims.

While various inventive aspects, concepts and features of the inventions may be described and illustrated herein as embodied in combination in the exemplary embodiments, these various aspects, concepts and features may be used in many alternative embodiments, either individually or in various combinations and sub-combinations thereof. Unless expressly excluded herein all such combinations and sub-combinations are intended to be within the scope of the present inventions. Still further, while various alternative embodiments as to the various aspects, concepts and features of the inventions--such as alternative materials, structures, configurations, methods, circuits, devices and components, alternatives as to form, fit and function, and so on--may be described herein, such descriptions are not intended to be a complete or exhaustive list of available alternative embodiments, whether presently known or later developed. Those skilled in the art may readily adopt one or more of the inventive aspects, concepts or features into additional embodiments and uses within the scope of the present inventions even if such embodiments are not expressly disclosed herein. Additionally, even though some features, concepts or aspects of the inventions may be described herein as being a preferred arrangement or method, such description is not intended to suggest that such feature is required or necessary unless expressly so stated. Still further, exemplary or representative values and ranges may be included to assist in understanding the present disclosure, however, such values and ranges are not to be construed in a limiting sense and are intended to be critical values or ranges only if so expressly stated. Parameters identified as "approximate" or "about" a specified value are intended to include the specified value, values within 5% of the specified value, and values within 10% of the specified value, unless expressly stated otherwise. Further, it is to be understood that the drawings accompanying the present disclosure may, but need not, be to scale, and therefore may be understood as teaching various ratios and proportions evident in the drawings. Moreover, while various aspects, features and concepts may be expressly identified herein as being inventive or forming part of an invention, such identification is not intended to be exclusive, but rather there may be inventive aspects, concepts and features that are fully described herein without being expressly identified as such or as part of a specific invention, the inventions instead being set forth in the appended claims. Descriptions of exemplary methods or processes are not limited to inclusion of all steps as being required in all cases, nor is the order that the steps are presented to be construed as required or necessary unless expressly so stated.

The invention further comprises the following embodiments:
1. A docking station frame for a medical device, the docking station frame comprising a plurality of strut portions extending from a proximal end to a distal end and forming a plurality of cells, wherein when the docking station frame is in an expanded, unconstrained condition, the plurality of strut portions form a substantially continuous curved longitudinal profile having outer radial retaining portions at the proximal end and the distal end and an inner radial central portion defining a valve seat.
2. The docking station frame of embodiment 1, wherein the plurality of strut portions comprise a plurality of circumferential strut portions axially spaced to define a plurality of rows of the plurality of cells, and a plurality of longitudinal strut portions circumferentially spaced and joined with the plurality of circumferential strut portions to define the plurality of cells of the plurality of cells in each of the plurality of rows.
3. The docking station frame of embodiment 2, wherein the plurality of circumferential strut portions comprises at least five circumferential strut portions defining at least four rows of the plurality of cells.
4. The docking station frame of any of embodiments 2 and 3, wherein the plurality of longitudinal strut portions comprises at least twelve longitudinal strut portions defining at least twelve cells in each of the plurality of rows.
5. The docking station frame of any of embodiments 2 - 4, wherein the plurality of circumferential strut portions comprises undulating strut portions.
6. The docking station frame of embodiment 5, wherein the undulating strut portions are substantially parallel and shaped such that each of the plurality of cells is substantially herringbone shaped.
7. The docking station frame of any of embodiments 5 and 6, wherein a distal one of the undulating strut portions defines the outer radial retaining portion at the distal end, and a proximal one of the undulating strut portions defines the outer radial retaining portion at the proximal end.
8. The docking station frame of any of embodiments 5 - 7, wherein each of the undulating strut portions includes proximal portions joined with a corresponding one of the plurality of longitudinal strut portions, and distal portions unattached from the plurality of longitudinal strut portions.
9. The docking station frame of any of embodiments 1 - 8, wherein when the docking station frame is in the expanded, unconstrained condition, the inner radial central portion has a diameter of about 27 mm and is expandable to accommodate a valve having a diameter of about 29 mm.
10. The docking station frame of any of embodiments 1 - 9, wherein when the docking station frame is in the expanded, unconstrained condition, the docking station frame has a length between about 35 mm and about 40 mm.
11. The docking station frame of any of embodiments 1 - 10, wherein when the docking station frame is in the expanded, unconstrained condition, the outer radial retaining portions extend at an angle between about 30° and about 45° with respect to a central longitudinal axis of the docking station frame.
12. The docking station frame of any of embodiments 1 - 11, wherein when the docking station frame is in the expanded, unconstrained condition, the inner radial central portion is substantially cylindrical.
13. The docking station frame of any of embodiments 1 - 12, wherein when the docking station frame is in the expanded, unconstrained condition, the outer radial retaining portions have a radius of curvature of between about 14 mm and about 20 mm.
14. The docking station frame of any of embodiments 1-13, wherein when the docking station frame is in the expanded, unconstrained condition, the inner radial central portion is contoured to configure the valve seat to have a length of at least about 8 mm for a valve having a deployed outer diameter of 29 mm.
15. The docking station frame of any of embodiments 1-14, wherein the docking station frame is configured to be crimped to a diameter of no greater than about 5.4 mm.
16. A docking station frame for a medical device, the docking station frame comprising a plurality of strut portions extending from a proximal end to a distal end and forming a plurality of cells, wherein when the docking station frame is in an expanded, unconstrained condition, the plurality of strut portions form outer radial retaining portions at the proximal end and the distal end extending at an angle between about 30° and about 45° with respect to a central longitudinal axis of the docking station frame.
17. A docking station frame for a medical device, the docking station frame comprising a plurality of strut portions extending from a proximal end to a distal end and forming a plurality of cells, wherein when the docking station frame is in an expanded, unconstrained condition, the plurality of strut portions form a curved longitudinal profile having outer radial retaining portions at the proximal end and the distal end and an inner radial central portion defining a valve seat, the inner radial central portion being substantially cylindrical and the outer radial retaining portions each having a radius of curvature between about 14 mm and about 20 mm.
18. A docking station for a medical device, the docking station comprising:
   the docking station frame of any of embodiments 1-17; and
   an impermeable material attached to the docking station frame.
19. A system comprising:
   a delivery catheter including a tube; and
   a docking station disposed in the tube, wherein the docking station comprises the docking station of embodiment 18.
20. A docking station for a prosthetic valve, the docking station comprising:
   an expandable docking station frame including a plurality of struts extending from a first end to a second end of the docking station frame, the expandable frame further defining a valve seat for securing a prosthetic valve to the expandable frame;
   wherein the first end of the docking station frame defines a retaining portion expandable radially outward of the valve seat to engage an inner surface of a circulatory system at a deployed position over a range of sizes of expansion; and
   a plurality of radiopaque markers disposed around the first end of the docking station frame.
21. A method of installing a prosthetic valve in a vessel, the method comprising:
   providing an expandable docking station frame in a compressed condition within a first tube;
   inserting the first tube into the vessel;
   deploying the docking station from the first tube and expanding the docking station frame at a target location within the vessel, such that a first end of the docking station frame expands radially outward of the valve seat to engage an inner surface of the vessel to retain the docking station frame at the target location;
   withdrawing the first tube from the vessel;
   providing an expandable prosthetic valve in a compressed condition within a second tube;
   inserting the second tube into the vessel;
   visually confirming a positional arrangement of the first end of the expanded docking station frame by visually identifying locations of a plurality of radiopaque markers disposed around the first end of the docking station frame;
   extending a terminal end of the second tube between the visually identified locations of the plurality of radiopaque markers and into the first end of the expanded docking station frame; and
   deploying the expandable prosthetic valve from the terminal end of the second tube and expanding the prosthetic valve into seating engagement with a valve seat of the expanded docking station frame.

## Claims

1. A docking station (10) for a prosthetic valve (29), the docking station comprising:
an expandable docking station frame (1500) including a plurality of struts (1501; 1502) extending from a first end (12) to a second end (14) of the docking station frame (1500), the expandable frame (1500) further defining a valve seat (18) for securing the prosthetic valve (29) to the expandable frame (1500);
wherein the first end (12) of the docking station frame (1500) defines a retaining portion (414) expandable radially outward of the valve seat (18) to engage an inner surface (416) of a circulatory system at a deployed position over a range of sizes of expansion; and
a plurality of radiopaque markers (1580) disposed around the first end (12) of the docking station frame (1500).

2. The docking station (10) of claim 1, further comprising one or more sealing portions (410) configured to provide a seal between the docking station (10) and an inner surface (416) of the circulatory system.

3. The docking station (10) of any of claims 1 or 2, further comprising an impermeable material (21) attached to the frame (1500).

4. The docking station (10) of claim 3, wherein the frame (1500) comprises a plurality of apices (1510), wherein the struts define a plurality of cells (1504); wherein the frame (1500) comprises a plurality of openings (1511) between the struts and the apices (1510) in portions of the frame (1500) which are not defined by the cells (1504).

5. The docking station (10) of claim 4, wherein the impermeable material (21) does not cover at least a portion of the openings (1511) at the first end (12) and/or the second end (14) of the frame (1500).

6. The docking station of any of claims 4 or 5, wherein the impermeable material (21) is configured to cover each cell (1504) and one-half of each opening (1511) at the first end (12) and the second end (14), respectively.

7. The docking station of any of claims 4 or 5, wherein the impermeable material (21) is configured to cover each cell (1504) and three-fourths of each opening (1511) at the first end (12) and to not cover any openings (1511) at the second end (14).

8. The docking station (10) of any of claims 1 to 7, wherein the radiopaque markers (1580) are integrated with the docking station.

9. The docking station (10) of any of claims 3 to 7, wherein the radiopaque markers (1580) are sewn into pouches in the impermeable material (21).

10. The docking station (10) of any of claims 1 to 9, wherein the frame (1500) has a concave profile extending from the retaining portions (414) that are flared to a narrower cylindrical concave waist portion (16) defining the valve seat (18).

11. The docking station (10) of any of claims 1 to 9, wherein the frame (1500) has an elongated cylindrical shape providing a uniform retaining and sealing profile portion having radially inward extending legs supporting the valve seat (18) radially inward of the retaining and sealing profile portion.

12. The docking station (10) of any of claims 1 to 9, wherein the frame (1500) has an hourglass shape with convex profiled end portions providing a retaining and sealing engagement with the vessel inner surface and a concave profiled waist portion (16) defining the valve seat (18).

13. The docking station (10) of any of claims 1 to 9, wherein the frame (1500) has an hourglass shape with flared profiled end portions providing a flared retaining engagement with the vessel inner surface, a concave profiled waist portion (16) defining the valve seat (18) and convex profiled sealing portions between the waist portion (16) and the flared profiled end portions.

14. The docking station (10) of any of claims 1 to 13, wherein the radiopaque markers (1580) include an aperture (1582) extending through a central portion of the marker (1580) such as to allow a suture to pass therethrough.

15. A system comprising:
a delivery catheter including a tube; and
a docking station disposed in the tube, wherein the docking station comprises the docking station of any of claims 1 to 14.
